# EUROPÄISCHE PATENTSCHRIFT

(11) **EP 1 804 736 B1**
(45) Veröffentlichungstag und Bekanntmachung des Hinweises auf die Patenterteilung: **14.12.2011**
(21) Anmeldenummer: 05802720.2
(22) Anmeldetag: 18.10.2005
(51) Int. Cl.: A61F 2/44

(54) **BANDSCHEIBENENDOPROTHESE MIT TRANSVERSAL BOGENFÖRMIG GEKRÜMMTEN ZYLINDRISCHEN ARTIKULATIONSFLÄCHEN FÜR DIE LENDEN- UND HALSWIRBELSÄULE**
INTERVERTEBRAL DISC ENDOPROSTHESIS HAVING CYLINDRICAL ARTICULATION SURFACES THAT ARE CURVED IN A TRANSVERSALLY ARCHED MANNER FOR THE LUMBAR VERTEBRAL COLUMN AND CERVICAL VERTEBRAL COLUMN
ENDOPROTHESE DE DISQUE INTERVERTEBRAL A SURFACES ARTICULAIRES CYLINDRIQUES CINTREES EN ARC DE CERCLE POUR PARTIE LOMBAIRE ET PARTIE CERVICALE DE LA COLONNE VERTEBRALE

(30) Priorität: 18.10.2004 WO PCT/DE2004/002333
(43) Veröffentlichungstag der Anmeldung: 11.07.2007
(73) Patentinhaber: Büttner-Janz, Karin, 12557 Berlin (DE)
(72) Erfinder: Büttner-Janz, Karin, 12557 Berlin (DE)
(74) Vertreter: Harrison, Robert John
(86) Internationale Anmeldenummer: PCT/DE2005/001886
(87) Internationale Veröffentlichungsnummer: WO 2006/042534

(56) Entgegenhaltungen:
- WO-A-2004/041131
- DE-U1- 20 320 454
- US-A- 5 258 031
- US-A- 5 401 269
- US-A1- 2003 208 273
- US-A1- 2004 199 253

## Beschreibung

Die Erfindung betrifft eine Bandscheibenendoprothese für den vollständigen Ersatz der Bandscheibe im Lenden- und Halswirbelsäulenbereich.

Die Idee des funktionserhaltenden künstlichen Bandscheibenersatzes ist zwar jünger als der endoprothetische Ersatz der Extremitätengelenke, jedoch inzwischen fast 50 Jahre alt [Büttner-Janz, Hochschuler, McAfee (Eds.): The Artificial Disc. Springer Verlag, Berlin, Heidelberg, New York 2003]. Sie resultiert aus biomechanischen Überlegungen, nicht zufrieden stellenden Ergebnissen von Versteifungsoperationen, Erkrankungen in der Nachbarschaft von Versteifungen und aus der Entwicklung neuer Materialien mit Langzeitbeständigkeit.

Mithilfe eines funktionserhaltenden Bandscheibenimplantats ist es möglich, eine Versteifungsoperation zu umgehen, d.h. die Bewegung im Zwischenwirbelraum zu erhalten bzw. wieder herzustellen. Durch die Implantation einer künstlichen Bandscheibe gelingt es auch, im in-vitro-Experiment die biomechanischen Eigenschaften des Bewegungssegments nach einer Nukleotomie weitgehend zu normalisieren.

Unterschieden werden Implantate zum Ersatz der gesamten Bandscheibe von solchen zum Ersatz des Nucleus pulposus. Implantate zum kompletten Bandscheibenersatz sind entsprechend voluminös; sie werden von ventral oder ventrolateral eingebracht. Eine Implantation im unmittelbaren Anschluss an eine standardmäßige Nukleotomie kann mit einer Prothese zum kompletten Bandscheibenersatz demzufolge nicht durchgeführt werden.

Die Indikation zum funktionserhaltenden Bandscheibenersatz umfasst, als Alternative zur operativen Fusion, neben der primären schmerzhaften Diskopathie auch voroperierte Patienten mit einem sogenannten Postdiskotomiesyndrom, Patienten, die einen wiederholten Bandscheibenvorfall in der gleichen Etage aufweisen und Patienten, die nach einer Versteifungsoperation eine Anschlusssymptomatik in einer Nachbarbandscheibe haben. Eine, wenn auch seltene Indikation, besteht zudem in einer Wechseloperation einer Bandscheibenendoprothese wegen Beschwerden des Patienten infolge einer Fehlplatzierung der bereits implantierten Prothese oder wegen der Zerstörung eines Prothesenteils. Als eine der Kontraindikationen wird kontrovers diskutiert die Arthrose der Wirbelbogengelenke.

Insgesamt werden derzeit über 10 verschiedene Prothesen zum totalen Bandscheibenersatz klinisch eingesetzt. Besonders bekannt sind bei der Lendenwirbelsäule die Charité Artificial Disc, die Prodisc, die Maverick, die FlexiCore und die Mobidisc (Übersicht in Clinica Reports, PJB Publications Ltd., Juni 2004) und bei der Halswirbelsäule die Bryan Prothese, die Prestige LP Prothese, die Prodisc-C und die PCM Prothese, welche im Folgenden beschrieben werden.

Die Prodisc Prothese für die Lendenwirbelsäule wird seit einer Weiterentwicklung zur Prodisc II seit 1999 implantiert. Es ist eine nach den Komponenten zwar 3-teilige, jedoch funktionell 2-teilige Bandscheibenprothese in der Gleitpaarung Metall-Polyethylen. Implantationen mit der Prodisc werden in der Lendenwirbelsäule und mit einem adaptierten Prothesenmodell, der Prodisc-C, auch in der Halswirbelsäule durchgeführt. Es stehen unterschiedliche Größen, Höhen (über den Polyethylenkern) und Lordosewinkel (über die Metall-Abschlussplatten) zur Verfügung. Das Vor- und Rückneigen sowie Rechts- und Linksneigen sind bei der Prothese in einem gleich großen Bewegungsumfang möglich, die Axialrotation wird konstruktionsgemäß nicht begrenzt.

Gleiches trifft zu für die beiden 2-teiligen Prothesen der Halswirbelsäule, die PCM Prothese in der Gleitpaarung Metall-Polyethylen und die Prestige LP Prothese in der Gleitpaarung Metall-Metall. Als Besonderheit weist die Prestige LP Prothese konstruktionsgemäß die Möglichkeit einer anterior-posterioren Translation auf, infolge der horizontal nach ventral verlängerten Konkavität, die im Frontalschnitt den gleichen Radius hat wie die Konvexität.

Die Maverick und die FlexiCore für die Lendenwirbelsäule sind funktionell 2-teilige Prothesen mit sphärischen konvex-konkaven Gleitpartnern, beide in einer Metall-Metall-Gleitpaarung. Die Mobidisc ist dagegen eine funktionell 3-teilige Prothese in der Gleitpaarung Metall-Polyethylen mit 2 Artikulationsbereichen. Der eine Bereich ist wie bei den vorgenannten 3 Prothesen ein Ausschnitt einer Kugel mit je einer konvexen und einer konkaven Fläche der artikulierenden Partner von gleichem Radius und der andere Bereich der Mobidisc ist plan. Obwohl im planen Bereich eine Abbremsung der Axialrotation vorgesehen ist, ist diese jedoch im konvex-konkaven Artikulationsbereich nicht limitiert. Dagegen weist die FlexiCore innerhalb der sphärischen Gleitflächen über einen schmalen Bereich eines Anschlags eine Rotationsbegrenzung auf.

Als kompakte Prothese für den kompletten Bandscheibenersatz der Halswirbelsäule ist die Bryan Prothese im klinischen Einsatz, die über konvexe Titanplatten mit poröser Oberfläche an den Wirbelkörpern fixiert ist und ihre biomechanischen Eigenschaften aus einem Polyurethan-Nucleus erhält.

Die längsten Erfahrungen mit totalem Bandscheibenersatz liegen mit der Charité Prothese vor, welche Gegenstand der DE 35 29 761 C2 und der US 5,401,269 ist. Diese Prothese wurde im Jahr 1982 von Dr. Schellnack und Dr. Büttner-Janz an der Berliner Charité entwickelt und später als SB Charité Prothese benannt. 1984 erfolgte die erste Operation. Die Bandscheibenprothese wurde weiterentwickelt und seit 1987 wird der aktuelle Typ dieser Prothese, Modell III, implantiert; inzwischen weltweit über 10000mal (DE 35 29 761 C2, US 5,401,269). Die Prothese ist funktionell 3-teilig in der Gleitpaarung Metall-Polyethylen in 2 gleichen sphärischen Gleitflächen, die zum einen der transversal sich bewegende Polyethylenkern aufweist, und zum anderen die entsprechend adaptierten konkaven Pfannen in den beiden Metall-Abschlussplatten. Für die Anpassung an die Anatomie des Zwischenwirbelraums stehen in der Fläche unterschiedliche Größen der Metallplatten der Charité Prothese und verschiedene Höhen der größenadaptierten Gleitkerne sowie winklige Prothesen-Abschlussplatten zur Verfügung, die, in sagittaler Richtung umgekehrt implantiert, auch als Wirbelkörperersatz dienen können. Die Primärverankerung der Charité Prothese erfolgt über 6 Zähnchen, die sich zu dritt leicht zur Mitte versetzt neben dem vorderen und hinteren konvexen Rand jeder Prothesenplatte befinden.

Die anderen Prothesen weisen bei den wirbelkörperseitigen Metallplatten andere Primärverankerungen auf, z.B. einen Kiel, der sagittal verläuft, eine strukturierte Oberfläche, eine konvexe Form mit z.B. quer verlaufenden Rillen und Kombinationen davon, auch mit unterschiedlich lokalisierten Zähnchen. Darüber hinaus können Verschraubungen zur Anwendung kommen, entweder von ventral oder von intern im Zwischenwirbelraum in den Wirbelkörper hinein.

Um die Verankerung der Prothesen-Abschlussplatten an den Wirbelkörpem langfristig zusätzlich zu gewährleisten und somit eine feste Verbindung mit dem Knochen zu erzeugen, wurde analog zu zementfreien Hüft- und Knieprothesen eine Oberfläche geschaffen, die Chrom-Kobalt, Titan und Kalziumphosphat so miteinander verbindet, dass Knochen direkt an die Abschlussplatten heranwachsen kann. Diese Verbindung zwischen Prothese und Knochen, ohne Ausbildung von Bindegewebe, ermöglicht eine lang andauernde Fixierung der künstlichen Bandscheibe und reduziert die Gefahr von Prothesenlockerungen, Verschiebungen der Prothese und Materialbrüchen.

Ein Hauptziel beim funktionserhaltenden Bandscheibenersatz besteht darin, die Bewegung zu erhalten und nach Möglichkeit die Bewegungsabläufe der Prothese weitgehend dem Bewegungsmuster einer gesunden Bandscheibe anzupassen. Im unmittelbaren Zusammenhang damit steht die Bewegung und Belastung in den Wirbelbogengelenken, die bei einer Fehlbeanspruchung ein eigenes Krankheitspotential haben. Es kann zu einer Abnutzung der Wirbelbogengelenke kommen (Arthrose, Spondylarthrose), im Vollbild mit der Ausbildung von Osteophyten. Durch diese Osteophyten und auch bei einem pathologischen Bewegungsmuster der Bandscheibe allein, ist die Reizung von Nervenstrukturen möglich.

Die gesunde Bandscheibe ist im Zusammenwirken mit den anderen Elementen des Bewegungssegments so aufgebaut, dass nur bestimmte Bewegungsumfänge möglich sind. So werden in der Bandscheibe zum Beispiel Vor- und Rückwärtsbewegungen des Rumpfes mit Drehbewegungen verbunden und auch Seitbewegungen kombiniert mit anderen Bewegungen ausgeführt. Die Bewegungsausschläge sind, bezogen auf die Extension (Rückneigen) und Flexion (Vorneigen) sowie das Seitneigen nach rechts und links und auch bezogen auf die Rotation, bei einer gesunden Bandscheibe im Ausmaß sehr unterschiedlich. Trotz übereinstimmender Grundmerkmale bestehen darüber hinaus Unterschiede in den Bewegungsausschlägen zwischen der Lenden- und Halswirbelsäule.

Bei Bewegungen in der Bandscheibe kommt es zu Veränderungen des Drehzentrums, d.h., die Bewegungen in der Bandscheibe erfolgen nicht um ein fixiertes Zentrum, sondern infolge einer simultanen Translationsbewegung der benachbarten Wirbel verändert das Zentrum stetig seine Lage (inkonstantes Rotationszentrum). Die Prothese nach der DE 35 29 761 C2 zeigt hierzu einen Aufbau, der sie von anderen verfügbaren Prothesentypen, welche wie ein Kugelgelenk aufgebaut sind und sich demzufolge nur um einen definiert lokalisierten Drehpunkt bewegen, unterscheidet. Durch den dreiteiligen Aufbau der Prothese nach der DE 35 29 761 C2 aus zwei metallischen Abschlussplatten und dem dazwischen liegenden, frei beweglichen Gleitkern aus Polyethylen wird der Bewegungsablauf der gesunden Bandscheibe in der humanen Wirbelsäule weitgehend nachempfunden, ausgenommen jedoch die exakten Bewegungsausschläge in die einzelnen Bewegungsrichtungen.

Ein weiteres wichtiges Merkmal der gesunden lumbalen Bandscheiben ist deren Trapezform, die für die Lordose der Lenden- und Halswirbelsäule hauptverantwortlich ist. Die Wirbelkörper selbst sind an der Lordose nur in geringem Ausmaß beteiligt. Bei einem endoprothetischen Ersatz der Bandscheiben sollte die Lordose möglichst erhalten bleiben bzw. rekonstruiert werden. Bei der Charité Bandscheibenprothese gibt es dafür vier verschieden gewinkelte Abschlussplatten, die zudem untereinander kombiniert werden können. Jedoch bedeutet es intraoperativ einen gewissen Aufwand und die Gefahr einer Schädigung der Wirbelkörperendplatten mit erhöhter Gefahr für ein Einsinken der Prothese in die Wirbelkörper, wenn nach der Implantation der Prothese diese komplett wieder entnommen werden muss, weil eine gute Lordoseeinstellung und Belastung des Polyethylenkernzentrums nicht erzielt werden konnten.

Um ein Abgleiten bzw. Herausrutschen des mittleren Gleitpartners aus den beiden Abschlussplatten zu verhindern, ist aus der DE 35 29 761 C2 ein Gleitkern mit einer zweiseitigen teilsphärischen Oberfläche (linsenförmig), mit einem planen Führungsrand und außen mit einer Ringwulst versehen, bekannt, der sich bei Extrembewegungen zwischen den beiden formadaptierten Abschlussplatten verklemmt. Auch aus der DE 102 42 329 A1 ist eine ähnliche Bandscheibenprothese bekannt, die um die Kontaktflächen herum eine Rille aufweist, in der ein mit der gegenüberliegenden Kontaktfläche in Kontakt befindlicher elastischer erster Ring zur besseren Führung eingebettet ist.

Die EP 0 560 141 B1 beschreibt eine 3-teilige Bandscheibenendoprothese, welche ebenfalls aus zwei Abschlussplatten und einem dazwischen lokalisierten Prothesenkern besteht. Die in dieser Druckschrift beschriebene Bandscheibenendoprothese setzt bei Drehung ihrer Abschlusslatten in entgegengesetzte Richtungen um eine vertikale Hochachse der Rotation ohne Anschläge an den Prothesenplatten einen Widerstand entgegen. Dies wird durch ein Aufgleiten der Abschlussplatten bei der Rotation auf den Prothesenkern durch das Gewicht, welches auf die Platten infolge der biomechanischen Lastübertragung in der Wirbelsäule einwirkt, erreicht, da sich im mittigen Sagittal- und Frontalschnitt die jeweiligen Krümmungsbögen voneinander unterscheiden.

Die vorstehenden Modelle sind als Implantate dauerhaft in den Bandscheibenräumen verankert. Es kann jedoch insbesondere bei zu kleinflächiger Lastübertragung mittel- bis langfristig zu einer Migration (Verschiebung) der Abschlussplatten in die Wirbelkörper hinein und somit zur Dislokation der gesamten Bandscheibenendoprothese kommen, wodurch artifizielle Belastungen der Wirbelkörper und der umgebenden Nerven und letztendlich des gesamten Bewegungssegments auftreten können, verbunden mit erneuten Beschwerden des Patienten. Zu diskutieren sind auch die Langzeitbeständigkeit des Polyethylens, wobei bereits Gleitkernzerstörungen in dreiteiligen Prothesen bekannt wurden mit daraus erforderlicher Reoperation, bisher als Versteifung des Bewegungssegments.

Wenn die Wirbelbogengelenke des Operationssegmentes bereits zur Prothesenimplantation eine Arthrose aufweisen, ist das Risiko postoperativ weiterbestehender Beschwerden erhöht. Ebenfalls zu beachten ist, dass ein zu großer segmentaler Bewegungsumfang infolge des Prothesendesigns unter Umständen zu erneuten Beschwerden des Patienten führen kann, am ehesten verursacht durch eine Über- oder Fehlbelastung der Wirbelbogengelenke, die eine schmerzhafte Arthrose entwickeln können. Gleiches trifft zu auf frontal winklig eingebaute oder postoperativ sich entwickelnde fehlstehende Bandscheibenendoprothesen. Dem gegenüber steht, dass eine Versteifungsoperation zur Mehrbelastung im Nachbarsegment führt mit der Gefahr einer späteren Operationsindikation in dieser Etage. Somit kann eine Bandscheibenendoprothese mit einer segmentalen Teilfunktion eine Problemlösung darstellen.

Die EP 1 039 855 B1 offenbart ein teilzylindrisches Implantat für den Wirbelkörperzwischenraum. Dieses Implantat weist einen elastischen Kern auf, der sich zwischen zwei Abschlussplatten befindet, welche mit einem oberen und unteren Wirbel verbunden werden. Eine Bewegung in dem Wirbelkörperzwischenraum ist nur in dem Maß möglich, wie sich der elastische Kern komprimieren lässt.

Auch die US 5,539,409 offenbart ein teilzylindrisches Implantat für den Wirbelkörperzwischenraum. Ein derartiges Implantat weist eine raue Oberfläche auf und soll erfindungsgemäß mit Substanzen gefüllt werden, welche eine Fusion des Implantats mit dem Knochenmaterial der benachbarten Wirbel fördert. Somit wird keine Bewegung in dem betroffenen Segment der Wirbelsäule nach Implantation mehr ermöglicht.

Des weiteren sind aus dem Stand der Technik noch Bandscheibenendoprothesen bekannt, welche eine oder mehrere zylindrische, komprimierbare Mittelteile aufweisen. So beispielsweise in der CA 2 376 097 A1, welche eine Prothese offenbart, die aus einer teilzylindrischen oberen und unteren Hülse besteht, zwischen der ein zylindrischer Mittelteil aus einem elastischen Material angeordnet ist.

Bei den aus dem Stand der Technik bekannten Bandscheibenendoprothese mit einem zylindrischen Kern, ist dieser zumeist aus einem elastischen Material gefertigt oder er dient der festen Verbindung der benachbarten Wirbelkörper. Die US 5,258,031 offenbart eine im seitlichen Querschnitt teilzylindrische Artikulationsfläche einer zweiteiligen Bandscheibenendoprothese, welche jedoch ein Seitneigen über die seitlichen Kanten der zylindrischen Randkonvexität zulässt, wodurch der auf den Abschlussplatten lastende Druck zeitweilig nur auf den Kanten lastet und somit ein erhöhter Verschleiß dieser Bereiche der Artikulationsflächen zu erwarten ist. Eine derartige Prothese kann wegen der großen Kiele oder Verschraubung zur Prothesenverankerung von ventral auch nur durch eine Operation von ventral eingebracht werden.

Die US 2003/0208273A1 (D1) offenbart eine Bandscheibenendoprothese, bei der ein erster Artikulationspartner mit einem ersten Wirbel verbunden werden kann und ein zweiter Artikulationspartner mit einem zweiten Wirbel. Eine Artikulationsbewegung zwischen den beiden Artikulationskomponenten wird durch aneinandergrenzende Artikulationsflächen in Form von Konvexität und Konkavität gewährleistet, wobei wenigstens eine der konvexen und konkaven Artikulationsoberflächen eine Furche in der Oberfläche aufweist, damit Dinge, die sich zwischen den Artikulationsflächen befinden leichter entfernt werden können.

Die WO 2004/041131A2 offenbart ein bewegliches Bandscheibenimplantat, welches aus drei oder mehr Teilen besteht. Dabei ist vorgesehen, dass die Teile des Implantates Bewegungen in verschiedene Richtungen und Ausmaße begrenzen können, um die natürlichen Bewegungen der Wirbelsäule zu imitieren. Dabei sind auch gerade zylindrische Artikulationsflächen vorgesehen. Nachteilig an einer Prothese gemäß der WO 2004/041131A2 ist, dass eine Rotation um die vertikale Körperachse ungebremst und im Zentrum der Prothese stattfindet. Ausgehend von diesem Stand der Technik ist es Aufgabe der vorliegenden Erfindung, eine Bandscheibenendoprothese für den totalen Bandscheibenersatz zur Verfügung zu stellen, welche eine dorsoventrale Bewegung und eine Rotationsbewegung eines Segmentes der Wirbelsäule ermöglicht, aber aufgrund der patientenspezifischen Indikation kein Seitneigen ermöglicht, wobei die Prothese auch durch eine Operation von ventrolateral oder von lateral eingebracht werden kann.

Gelöst wird diese Aufgabe durch die Merkmale der selbstständigen Ansprüche 1 und 2. Die Erfindung sieht zwei unterschiedliche Arten einer Bandscheibenendoprothese vor, nämlich eine funktionell zweiteilige und eine funktionell dreiteilige Prothese.

Die funktionell zweiteilige Prothese nach Anspruch 1 zeichnet sich dadurch aus, dass
a) ein erster Gleitpartner derart ausgebildet ist, dass die zur Verbindung mit einem Wirbelkörper entgegengesetzte Seite eine konvexe Krümmung (Konvexität) aufweist, und die Geometrie der Konvexität dadurch definiert ist, dass diese einem Abschnitt eines Zylinders entlang seiner Längsachse von rechts nach links lateral mit einer transversal nach ventral gerichteten bogenförmigen Krümmung entspricht, wobei die Konvexität dorsal, ventral und beidseits lateral von einem Rand abgeschlossen wird, und
b) ein zweiter Gleitpartner derart ausgebildet ist, dass die zur Verbindung mit einem Wirbelkörper entgegengesetzte Seite mit einer konkaven Artikulationsfläche (Konkavität) ausgebildet ist und die Geometrie der Konkavität dadurch definiert ist, dass diese eine zur Konvexität des ersten Gleitpartners korrespondierende Ausnehmung aufweist, und die Konkavität dorsal, ventral und beidseits lateral von einem Rand abgeschlossen wird, und
c) die Ränder beider Gleitpartner einen sich nach außen öffnenden Winkel (Öffnungswinkel) zueinander aufweisen, wobei
   a. keine Neigung der Gleitpartner zueinander in lateraler Richtung möglich ist, und
   b. die maximal mögliche Bewegung der Gleitpartner in dorsoventraler Richtung durch einen Lückenschluss der Ränder der beiden Gleitpartner begrenzt wird, und
d) die Rotation der Gleitpartner zueinander begrenzt wird durch die Toleranz zwischen Konvexität und Konkavität rechts und links lateral der transversal gekrümmten zylindrischen Artikulationsflächen.

Die funktionell dreiteilige Prothese nach Anspruch 2 zeichnet sich dadurch aus, dass
a) der mittlerer Gleitpartner auf Ober- und Unterseite eine konvexe Krümmung (Konvexität) aufweist, und die Geometrie der Konvexitäten dadurch definiert ist, dass diese einem Abschnitt eines Zylinders entlang seiner Längsachse von rechts nach links lateral mit einer transversal nach ventral gerichteten bogenförmigen Krümmung entspricht, und
b) oberer und unterer Gleitpartner mit einer konkaven inneren Artikulationsfläche (Konkavität) ausgebildet sind und die Geometrie der Konkavitäten dadurch definiert ist, dass diese eine zur artikulierenden Konvexität des mittleren Gleitpartners korrespondierende Ausnehmung aufweist, und die Konkavitäten auf Ober- und Unterseite jeweils dorsal, ventral und beidseits lateral von einem Rand abgeschlossen werden, und
c) die Ränder der artikulierenden Gleitpartner einen sich nach außen öffnenden Winkel (Öffnungswinkel) zueinander aufweisen, wobei
   a. keine Neigung der Gleitpartner zueinander in lateraler Richtung möglich ist, und
   b. die maximal mögliche Bewegung der Gleitpartner in dorsoventraler Richtung durch einen Lückenschluss der Ränder der Gleitpartner begrenzt wird, und
d) die Rotation der Gleitpartner zueinander begrenzt wird durch die Toleranz zwischen Konvexität und Konkavität rechts und links lateral der transversal gekrümmten zylindrischen Artikulationsflächen.

Beiden Prothesen ist gemeinsam, dass sie aus artikulierenden Gleitpartnern bestehen, von denen der jeweils obere Gleitpartner fest mit einem oberen Wirbelkörper und der jeweils untere Gleitpartner fest mit einem unteren Wirbelkörper verbunden ist, und wobei die Gleitpartner auf ihren zueinander gerichteten Innenseiten mit ineinandergreifenden Artikulationsflächen ausgebildet sind. Oberer und unterer Gleitpartner einer dreiteiligen Prothese sowie die beiden Gleitpartner einer zweiteiligen Prothese fungieren gleichzeitig als Abschlussplatten, welche Mittel aufweisen, die zur Verbindung mit einem oberen bzw. unteren Wirbelkörpern dienen.

Die bogenförmige Krümmung der zylindrischen Konvexitäten und korrespondierenden Konkavitäten ist nur in einem geringen Maße vorgesehen. Durch die nach ventral bogenförmige Krümmung der Artikulationsflächen der Prothese wird neben einer Bewegung nach ventral und dorsal eine weitere Teilfunktion einer natürlichen Bandscheibe realisiert, die Rotation. Die Wirbelbogengelenke befinden sich im menschlichen Körper in etwa auf dem Kreisbogen bzw. zirkulär parallel davon, der von der bogenförmigen Krümmung der Artikulationsflächen anteilig gebildet wird, mit dadurch besonders vorteilhafter biomechanischer Beeinflussung der Wirbelbogengelenke während der Rotation. Die Rotation der Gleitpartner zueinander dient unter auch einer Schonung des Materials der Prothese und der Knochen-Implantatgrenze, woraus eine geringere Beanspruchung entsteht.

Erfindungsgemäß ist vorgesehen, dass der ventrale und dorsale Krümmungsradius der zylindrischen Konvexität und korrespondierenden Konkavität in einer transversalen Aufsicht in konstantem Abstand zueinander verlaufen, da die ventrale und dorsale Krümmung von zwei Kreisen mit unterschiedlichen Radien, aber identischen Mittelpunkten, abgeleitet ist. Es ist erfindungsgemäß aber auch vorgesehen, dass die Kreisbögen nicht in einem konstanten Abstand zueinander verlaufen und entweder nach lateral aufeinander zulaufen oder nach lateral einen zunehmenden Abstand aufweisen. Im ersten Fall würden sich Konvexität und korrespondierende Konkavität nach lateral jeweils verjüngen, wodurch mehr "Spiel" für die kombinierte Rotations- und Neigungsbewegung entsteht. Im zweiten Fall würde die Kontaktfläche der Konvexität mit der Konkavität vergrößert sein mit dadurch besserer Materialbeständigkeit der Artikulationsflächen.

Insgesamt wird demnach bei einer erfindungsgemäßen Bandscheibenendoprothese die maximal mögliche Bewegung der Gleitpartner
a) bei der dorsoventralen Neigung der Gleitpartner zueinander durch den Krümmungsradius sowie die Höhe der Konvexität und Konkavität und des jeweiligen Randes, der eine Artikulationsfläche ventral und dorsal umgibt, und
b) den Öffnungswinkel zwischen den Rändern benachbarter Gleitpartner, wobei dieser durch schräg und/oder waagerecht verlaufende Ränder der benachbarten Gleitpartner gebildet wird, wobei es bei endgradigem Kontakt der Gleitpartner zu einem Lückenschluss kommt, und
c) durch die Rotation der Gleitpartner in Bezug auf eine gedachte Vertikalachse, durch die bogenförmige(n) Krümmung(en) und das Ausmaß der bogenförmigen Krümmung(en) der zylindrischen Konvexität(en) und der korrespondierenden Konkavität(en) und die Toleranz zwischen Konvexität und Konkavität jeweils rechts und links lateral am Ende der zylindrischen Artikulationsflächen festgelegt.

Die zwei- und dreiteilige Prothese sind besonders vorteilhaft bei Prothesenimplantationen in mehrere übereinander befindliche Bandscheiben wegen der modellimmanenten Stabilität. Sie sind auch vorteilhaft bei nach rechts oder links gewinkelten Bandscheibenräumen, die es zu korrigieren gilt. Darüber hinaus können die zwei- und dreiteiligen Prothesen bei Patienten eingesetzt werden, denen von lateral oder ventrolateral eine Prothese implantiert werden soll, z.B. auch über einen Transpsoaszugang. Eine derartige Indikation ist gegeben bei z.B. an der Wirbelsäule von ventral voroperierten Patienten und bei Patienten, die einer Prothesenwechseloperation nach primärer Prothesenimplantation von ventral bedürfen, da die Vernarbung der großen Blutgefäße im ventralen Wirbelsäulenbereich bei einer ventralen Reoperation ein erheblich erhöhtes Operationsrisiko darstellt. Beim seitlichen Zugang kann der Faserring einseitig großzügig inzidiert werden, um eine in der Fläche optimale Prothese für eine großflächige Druckübertragung zu positionieren, ohne dass es postoperativ infolge der einseitig lateralen Bandscheibenliberation zur frontal segmentalen Winkelstellung des Zwischenwirbelraums kommt. Weiterhin ist denkbar, dass mit der Prothese auch Patienten behandelt werden können, die eine osteophytenfreie Arthrose der Wirbelbogengelenke aufweisen, da postoperativ keine Seitneigebewegung diese Gelenke belastet.

Die zweiteilige Prothese ist besser geeignet für die Halswirbelsäule wegen der engen anatomischen Bedingungen in dieser Region der Wirbelsäule. Gegenüber der zweiteiligen Prothese hat die dreiteilige Bandscheibenendoprothese den Vorteil, dass das Transversalgleiten zweier benachbarter Wirbel nur minimal ist, mit dadurch hervorgerufener besonders vorteilhafter Adaptation an die Biomechanik des Bewegungssegments der Lendenwirbelsäule. Mit der dreiteiligen Prothese kann zudem das inkonstante Rotationszentrum simuliert werden.

Im Zusammenhang mit der vorliegenden Erfindung werden die drei Körperachsen durch die folgenden Begriffe bezeichnet: Ein Sagittalschnitt oder eine Ansicht in der sagittalen Ebene erlaubt eine Seitenansicht, da die zugrunde liegende Schnittebene senkrecht von vorn nach hinten verläuft. Für die Angabe "vorn" wird auch "ventral" Angabe und für die "hinten" analog "dorsal" verwendet, da dies die Orientierung einer Prothese im Körper anzeigt. Ein "Frontalschnitt" oder die "frontale Ebene," ist ein senkrechter Querschnitt von einer Seite zur anderen Seite. Für die Angabe "seitlich" wird auch der Begriff "lateral" verwendet. Sowohl Sagittal- als auch Frontalschnitt sind Vertikalschnitte, da sie entlang einer vertikalen Ebene verlaufen, jedoch um 90 Grad versetzt zueinander orientiert sind. Eine Ansicht in "transversaler Ebene" oder ein "Transversalschnitt" erlaubt eine Aufsicht auf die Prothese, da es sich um einen Horizontalschnitt handelt.

In Verbindung mit der Beschreibung und Darstellung der vorliegenden Erfindung wird unter einer Artikulationsfläche der Bereich von Gleitpartnern verstanden, der aus den gekrümmten konvexen und konkaven Teilen der Oberflächen besteht, welche in Kontakt kommen und miteinander oder aufeinander gleiten bzw. artikulieren. Aus diesem Grund wird für Artikulationsfläche auch gleichbedeutend die Bezeichnung Gleitfläche verwendet.

Der Begriff korrespondierend bezeichnet im Zusammenhang mit artikulierenden Gleitflächen nicht ausschließlich kongruente konvexe und konkave Flächen, die miteinander artikulieren. Vielmehr werden damit auch miteinander artikulierende Gleitflächen bezeichnet, deren Oberflächen nicht vollständig kongruent sind. Derartige "Abweichungen" bzw. Toleranzen bezüglich der Gleitflächen artikulierender Gleitpartner können zum Einen durch die gewählten Materialien und Formen bedingt sein. Andererseits kann es aber auch beabsichtigt sein, dass die Gleitflächen nicht vollständig kongruent sind, um beispielsweise die zueinander gewünschten Bewegungsmöglichkeiten der Artikulationspartner gezielt vorgeben zu können.

Neben den Vorteilen, die sich aus der erfindungsgemäßen Formgebung der bogenförmig gekrümmten konvex-konkaven Teile der Artikulationsflächen ergeben, weisen die erfindungsgemäßen Bandscheibenendoprothesen noch weitere Vorteile auf. Bei einer zwei- und dreiteiligen erfindungsgemäßen Bandscheibenendoprothese sind die Konkavitäten von oberem und unterem Gleitpartner jeweils von einem Rand umschlossen, wohingegen sich die Konvexitäten des mittleren Gleitpartners einer dreiteiligen Prothese jeweils über die gesamte Ober- und Unterseite erstrecken, d.h. die Konvexitäten randfrei sind, oder die Konvexitäten jeweils von einem Rand umgeben sind, dessen Breite gleich oder unterschiedlich ist.

Unter einem Rand soll im Sinne der vorliegenden Erfindung eine Fläche verstanden werden, welche sich zwischen Außenkante des jeweiligen Gleitpartners und Konvexität(en) bzw. Konkavität(en) befindet. Die Ränder der jeweiligen Gleitpartner verlaufen waagerecht und/oder schräg und weisen vorzugsweise eine plane Oberfläche auf. Wesentlich für die Gestaltung der Oberfläche der Ränder ist es, dass es bei einer endgradigen Neigung der Gleitpartner zueinander, zu einem möglichst großflächigen Lückenschluss zwischen den Rändern der Gleitpartner kommt. Sofern die Ränder keine plane Oberfläche aufweisen, sind diese jedenfalls so gestaltet, das es bei einem Lückenschluss zu einem möglichst großflächigen Kontakt der Ränder kommt. Die Höhe der Ränder im unmittelbaren Übergangsbereich der artikulierenden Fläche zur Fläche des Randes entlang der Artikulationsflächen ventral bzw. dorsal ist gleich oder unterschiedlich gestaltet, jedoch kann die Randhöhe ventral zu dorsal so unterschiedlich sein, dass die Bewegungsmöglichkeit nach ventral gezielt größer ist als nach dorsal.

Die Ränder der konvexen und konkaven Artikulationsfläche(n) weisen ohne Neigung der Gleitpartner zueinander in jeder vertikalen Schnittebene immer einen nach außen offenen Winkel (Öffnungswinkel) auf. Bei einer endgradigen Neigung der Gleitpartner zueinander kommt es zwischen den Rändern der Artikulationsflächen zu einem Lückenschluss. Die maximalen Neigungswinkel werden durch Kontakt des Übergangs zwischen konvexer und konkaver Artikulationsfläche und der Fläche des Randes begrenzt. Diese Kontaktzone ist zwar limitierend für die weitere Bewegung der Gleitpartner zueinander, sie ist aber nicht der einzige Bereich außerhalb des konkav-konvexen Teils der Artikulationsflächen, welcher bei endgradiger Neigung in Kontakt kommt. So sind die Ränder der Gleitpartner bis zu deren äußerer Begrenzung derart ausgestaltet, dass diese ebenfalls an dem Lückenschluss beteiligt sind.

Durch diese erfindungsgemäße Maßnahme wird die Fläche vergrößert, auf welche sich der Druck verteilen kann, der bei einer Neigung der Prothese bis zum Anschlag auftritt. Da dieser Druck durch einen flächigen Kontakt aufgenommen wird und nicht durch kleine Kontaktzonen, werden die miteinander in Kontakt befindlichen Oberflächen zusätzlich vor Abnutzung geschützt, wodurch die Prothese deutlich langlebiger wird.

Ferner ist erfindungsgemäß vorgesehen, dass eine zylindrische Konvexität entlang ihrer Längsachse sich einseitig konisch verringernde Radien aufweist. In einer derartigen Ausführungsform weist der Rand eine entsprechende Anpassung auf, so dass weiterhin die ventrodorsale und rotierende Bewegung möglich sind. Durch diesen "schiefen" Zylinder ist es möglich, Anpassungen an eine Fehlstellung des Wirbelkörperzwischenraums vorzunehmen und/oder eine vorhandene Skoliose zu erhalten oder - sofern indiziert - auszugleichen.

Bezüglich des Materials ist bei einer erfindungsgemäßen Bandscheibenendoprothese vorgesehen, dass die Gleitpartner einstückig ausgebildet sind oder wenigstens ein Gleitpartner aus zwei fest oder fest, aber reversibel miteinander verbundenen Teilen besteht, wobei entweder die Konvexität(en) und/oder Konkavität(en) den Teil ausmachen, der fest oder fest, aber reversibel mit dem jeweiligen Gleitpartner verbunden ist, oder Konvexität(en) und/oder Konkavität(en) an der Basis geeignete Mittel für eine feste oder feste, aber reversible Verbindung mit dem jeweiligen Gleitpartner aufweisen, wobei die miteinander verbundenen Teile aus gleichen oder verschiedenen Materialien bestehen oder die Oberflächen der miteinander artikulierenden Flächen gleich oder verschieden beschichtet sind. Als geeignete Mittel für eine Verbindung sind erfindungsgemäß Anpassungen der Form der miteinander verbundenen Teile oder der der Konvexität oder Konkavität entgegengesetzten Seite, wie beispielsweise flächige Verbreiterungen, die Teil des Randes oder der gesamte Rand sind, oder Ausnehmungen, vorgesehen.

Sofern eine erfindungsgemäße Bandscheibenendoprothese aus fest oder fest, aber reversibel miteinander verbundenen Teilen besteht, ist für die Verbindung zwischen Gleitpartner und Konvexität(en) oder Konkavität(en) vorzugsweise eine Nut/Feder-Verbindung, eine Führungsschiene und korrespondierende Ausnehmung, ein Schnappmechanismus, Kleben oder Verschrauben vorgesehen.

Bei einer dreiteiligen erfindungsgemäßen Bandscheibenendoprothese ist erfindungsgemäß auch vorgesehen, dass oberer und unterer Gleitpartner aus dem gleichen Material bestehen oder gleich beschichtet sind und der mittlere Gleitkörper aus einem anderen Material gefertigt oder anders beschichtet ist.

Die Gleitpartner werden aus in der Implantattechnik bewährten Materialien gefertigt; beispielsweise bestehen der obere und untere Gleitpartner aus nichtrostendem Metall und der mittlere Gleitpartner aus medizinischem Polyethylen. Andere Materialkombinationen sind denkbar. Die Verwendung anderer alloplastischer Materialien, die auch bioaktiv oder auch stumpf sein können, ist ebenfalls denkbar. Die Gleitpartner sind an den zueinander gerichteten Berührungsflächen hochglanzpoliert, um den Abrieb zu minimieren (low-friction-Prinzip). Im übrigen ist auch eine Beschichtung der einzelnen Gleitpartner mit geeigneten Materialen vorgesehen. Bevorzugt sind folgende Materialien vorgesehen: Titan, Titanlegierungen oder Titancarbid, Legierungen aus Kobalt und Chrom oder anderen geeigneten Metallen, Tantal oder geeignete Tantalverbindungen, geeignete keramische Materialien sowie geeignete Kunststoffe oder Verbundwerkstoffe.

Ferner ist bei einer erfindungsgemäßen dreiteiligen Prothese vorgesehen, dass die Krümmungsradien der zylindrischen Konvexitäten auf Ober- und Unterseite des mittleren Gleitpartners sowie die korrespondierenden Konkavitäten in oberem und unterem Gleitpartner identisch oder unterschiedlich sind. Sowohl bei identisch als auch bei unterschiedlich gekrümmten Konvexitäten auf Ober- und Unterseite ist abhängig von der Ausführungsform ferner vorgesehen, dass die maximale Höhe der Konvexitäten des mittleren Gleitpartners auf Ober- und Unterseite unterschiedlich ist, so dass die Konvexitäten in Sagittalansicht aus peripheren Kreisabschnitten bestehen, die sich nicht zu einem vollständigen Kreisbogen verbinden lassen. Abhängig von der Ausführungsform ist die Höhe des Randes eines mittleren Gleitpartners um einen gleichen Betrag wie die Höhe der Konvexität(en) verringert, oder die Höhe des Randes gleich bleibt oder different zur Höhenänderung der Konvexität(en) ist, wobei die maximale Höhe der Konvexitäten auf Ober- und Unterseite dadurch gleich oder unterschiedlich ist.

Durch diese erfindungsgemäße Maßnahme wird die Gesamthöhe der Prothese reduziert, da der mittlere Gleitpartner abgeflacht ist. Dadurch werden Maße bei einer derart ausgestalteten Prothese erreicht, die es auch ermöglichen, diese in physiologisch besonders schmale Bandscheibenräume einzusetzen. Zudem bietet eine derartige Ausführungsform die Möglichkeit, die Höhe des mittleren Gleitpartners zu variieren und so eine in der Höhe angepasste Prothese zu erhalten.

Bei einer erfindungsgemäßen zwei- oder dreiteiligen Bandscheibenendoprothese ist jeweils ein maximaler Öffnungswinkel bei einseitigem Lückenschluss der Gleitpartner bei Extension oder Flexion zwischen 6 und 10 Grad vorgesehen. Adaptiert an die Lenden- bzw. Halswirbelsäule können die konkreten maximalen Bewegungsmaße konstruktiv angepasst werden, ohne für jede einzelne Bandscheibe eine "eigene Prothese" vorsehen zu wollen. Die dorsalen und ventralen Öffnungswinkel werden durch eine geeignete Wahl der Konvexitäten und Konkavitäten in Zusammenhang mit der Ausgestaltung der diese umgebenden Ränder erreicht. Dadurch kann eine nach ventral größere Neigung der Gleitpartner zueinander ermöglicht werden als nach dorsal, was der physiologischen Situation in der Lendenwirbelsäule entspricht. Zum Ausgleich von Toleranzen im Bewegungssegment werden zusätzliche 3 Grad in jede Bewegungsrichtung einbezogen.

Sowohl bei einer funktionell zwei- als auch bei einer funktionell dreiteiligen Bandscheibenendoprothese wird bei einer transversal nach ventral gerichteten bogenförmigen, zylindrischen Konvexität und Konkavität artikulierender Gleitpartner eine Rotation um eine gedachte zentrale Vertikalachse sowohl ermöglicht als auch abgebremst. Durch diese erfindungsgemäße Ausführungsform wird eine begrenzte Rotationsbewegung der Gleitpartner zueinander ermöglicht, die abhängig von dem Ausmaß der bogenförmigen Krümmung eine Rotation um eine gedachte zentrale Vertikalachse von bis zu 3 Grad für die Lendenwirbelsäule und bis zu 6 Grad für die Halswirbelsäule nach jeder Seite ermöglicht. Zum Ausgleich von Toleranzen im Bewegungssegment werden zusätzliche 2 Grad nach jeder Seite einbezogen.

In einer weiteren bevorzugten Ausführungsform einer erfindungsgemäßen zwei- oder dreiteiligen Bandscheibenendoprothese ist ein "Versatz" von Konvexität(en) und korrespondierenden Konkavität(en) um bis zu 4 mm vom mittigen Sagittalschnitt nach dorsal vorgesehen.

Ein nach dorsal versetztes Rotationszentrum entspricht vor allem der physiologischen Situation im Übergang zwischen der Lendenwirbelsäule und dem Kreuzbein und andererseits werden so parallel die der physiologischen Situation entsprechenden Unterschiede zwischen den möglichen Neigungswinkeln bei der Extension und Flexion erreicht.

Ferner ist vorgesehen, dass die Ränder der Gleitpartner ventral, dorsal und lateral rechtwinklig, anderweitig winklig, gekrümmt oder kombiniert gerade, gekrümmt und/oder winklig abgeschlossen sind. Bei einer dreiteiligen Prothese ist zudem vorgesehen, dass die Konvexitäten des mittleren Gleitpartners im äußeren, beidseits lateralen Bereich symmetrisch oder asymmetrisch, rechtwinklig, anderweitig winklig, halbkugelförmig, abgerundet oder abgeflacht abschneiden. Die artikulierende Konkavität weist eine zur lateralen Ausgestaltung der Konvexität korrespondierende Form auf. Der mittlere Gleitpartner wird auch bei endgradiger Neigung noch zwischen dem oberen und unteren Gleitpartner verbleiben und dadurch wird eine sehr kompakte und platzsparende Bauweise einer erfindungsgemäßen Bandscheibenendoprothese ermöglicht.

Bei dieser "kompakten" Ausführungsform einer erfindungsgemäßen dreiteiligen Bandscheibenendoprothese wird ein Herausgleiten des mittleren Gleitpartners einerseits durch die Höhen der Konvexität auf Ober- und Unterseite und der korrespondierenden Konkavitäten ab dem Rand der Artikulationsflächen und andererseits durch den Lückenschluss der Ränder der Gleitpartner bei endgradiger Neigung verhindert. Die Konvexitäten sind derart gestaltet, dass sie tief genug in die artikulierenden Konkavitäten "eingreifen". Ein ausreichendes Aufspreizen der gesamten Prothese postoperativ, welches für das Herausgleiten des mittleren Gleitpartners Voraussetzung wäre, ist somit nicht möglich.

Weiterhin ist es erfindungsgemäß vorgesehen, dass bei einem mittleren Gleitpartner einer dreiteiligen Prothese zur zusätzlichen Sicherung gegen ein Herausgleiten, Abgleiten bzw. Herausrutschen (Luxation) bei Lückenschluss aller drei Gleitpartner, ein Anschlag Teil des Randes des mittleren Gleitpartners ist, der außerhalb des oberen und/oder unteren Gleitpartners angeordnet ist, wobei der Anschlag wenigstens auf Ober- oder Unterseite höher ist als der Rand des mittleren Gleitpartners ist.

Dieser Anschlag zur zusätzlichen Sicherung gegen ein Herausgleiten, Abgleiten bzw. Herausrutschen (Luxation) kann erfindungsgemäß auch derart ausgestaltet sein, dass der Anschlag Teil des Randes des mittleren Gleitpartners ist, wobei dieser auf Ober- und/oder Unterseite höher als der Rand des mittleren Gleitpartners ist und innerhalb einer Nut im Randbereich des oberen und/oder unteren Gleitpartners mit dem notwendigen Spiel für die maximale Gleitbewegung der Gleitpartner geführt wird.

Unter einem Anschlag soll im Sinne der vorliegenden Erfindung eine nach außen gerichtete Fortführung des Randes eines mittleren Gleitpartners verstanden werden, welche aufgrund der jeweiligen Ausgestaltung geeignet ist, ein Herausgleiten des mittleren Gleitpartners aus den Konkavitäten des oberen und unteren Gleitpartners zu verhindern. Ein Anschlag muss den mittleren Gleitpartner dazu nicht vollständig umschließen, da dies zu Einschränkungen der maximalen Beweglichkeit aller Gleitpartner führen kann, sondern gegebenenfalls in definierten Abständen oder gegenüber von Positionen des Randes angeordnet sein, welche für ein Herausgleiten des mittleren Gleitpartners in Frage kommen. Sofern der Anschlag auf Ober- und Unterseite höher als der Rand des mittleren Gleitpartners ist, kann er beispielsweise wie eine Heftzwecke ausgestaltet sein, die mit der Nadelspitze von außen in den Rand des mittleren Gleitpartners gesteckt wurde, so dass der Kopf der Heftzwecke oben und unten über den Rand des mittleren Gleitpartners übersteht und bei einer endgradigen Neigung zur Position der Heftzwecke das Herausgleiten des mittleren Gleitpartners verhindert, indem er an dem oberen und unteren Gleitpartner "anschlägt".

Ist ein Anschlag zur Sicherung gegen ein Herausgleiten Teil des Randes der Gleitpartner, so ist die Höhe der Konvexität unter Beachtung der Anatomie und Materialeigenschaften lediglich abhängig von den gewünschten maximalen Neigungswinkeln, auf welche diese auch Einfluss hat (s.o.).

Ein Anschlag zur Sicherung des mittleren Gleitpartners bei einer dreiteiligen Prothese ist vorteilhafterweise derart gestaltet, dass er bei einer endgradigen Neigung der Gleitpartner ebenfalls an dem Lückenschluss des Randes beteiligt ist. Dadurch kommt dem Anschlag nicht nur eine Sicherungsfunktion zu, sondern er dient zusätzlich der Vergrößerung der mit Druck belasteten Flächen im Falle der endgradigen Neigung der Gleitpartner, deren Vorteile bereits beschrieben wurden. Die Möglichkeit einer derartigen Gestaltung hängt aber entscheidend von der Außenform des oberen und unteren Gleitpartners und der jeweiligen Randbreite von Konvexität und Konkavität ab.

In einer weiteren Ausführungsform einer dreiteiligen Bandscheibenendoprothese ist vorgesehen, dass die Höhe des Randes vom mittleren Gleitpartner ab dem Übergangsbereich zwischen der Konvexität und dem Rand bis zum äußeren Randbereich teilweise oder insgesamt kontinuierlich größer wird, ohne dass sich die Größe der Öffnungswinkel infolge Anpassung der Randhöhe des oberen und unteren Gleitpartners ändert. Diese "Schwalbenschwanzform" vom Rand des mittleren Gleitpartners erhöht dessen Sicherheit entgegen einer Dislokation.

Erfindungsgemäß ist bei dreiteiligen Prothesen auch eine Form von oberem und/oder unterem Gleitpartner vorgesehen, bei dem äußere Randbereiche vollständig oder partiell hakenförmig, rechtwinklig, anderweitig winklig, gekrümmt oder in Kombinationen davon in Richtung des anderen äußeren Gleitpartners ausgestaltet sind. Der Rand des mittleren Gleitpartners ist dort bei dieser Ausführungsform schmaler, so dass der mittlere Gleitpartner partiell oder vollständig von den Vorrichtungen eines oder beider äußerer Gleitpartner eingefasst wird, um ein Herausgleiten des mittleren Gleitkörpers zu verhindern. Vorteilhafterweise ist der Rand des mittleren Gleitpartners derart an die Randform eines äußeren Gleitpartners angepasst, dass bei einem endgradigen Lückenschluss eine möglichst große Fläche der artikulierenden Gleitpartner in Kontakt kommt.

Ferner ist bei der erfindungsgemäßen Bandscheibenendoprothese vorgesehen, dass sich der Außenumfang des oberen und unteren Gleitpartners in transversaler Ansicht von dorsal nach ventral (Lendenwirbelsäule) oder von ventral nach dorsal (Halswirbelsäule) verjüngen kann. Diese Verjüngung des Außenumfangs des oberen und unteren Gleitpartners kann lateral jeweils als identische Krümmung ausgebildet sein und ist bevorzugt ein Teilausschnitt eines Kreises. Fläche und Form des Außenumfanges des oberen und unteren Gleitpartners können je nach Bedarf gleich oder ungleich sein und so an die jeweilige Größe des Wirbelkörpers, mit dem sie verbunden werden, angepasst werden.

Die sich verjüngende Form der oberen und unteren Gleitpartner entspricht im wesentlichen der für die Prothesenplatten nutzbaren Fläche eines Wirbelkörpers in der Transversalansicht und führt so zu einer optimalen Nutzung der zur Verfügung stehenden Fläche eines Wirbelkörpers zur Verankerung der Gleitpartner mit dem Ziel einer möglichst großflächigen Lastübertragung des auf den Gleitpartnern aufliegenden Druckes.

Ferner sind bei einer erfindungsgemäßen Bandscheibenendoprothese Gleitpartneranpassungen vorgesehen, wobei oberer und/oder unterer Gleitpartner im Frontal- und/oder Sagittalschnitt derart ausgebildet sind, dass die Außen- und Innenseite von oberem und/oder unterem Gleitpartner parallel oder eben nicht parallel zueinander verlaufen. Durch diese erfindungsgemäße Maßnahme kann eine erfindungsgemäße Bandscheibenendoprothese an Wirbelkörperendplatten angepasst werden, welche in Frontalansicht nicht parallel zueinander stehen bzw. in der Sagittalansicht eine optimale Lordose und Gleitflächenstellung zueinander ausbilden sollen.

Weiterhin ist vorgesehen, dass bei einer zwei- und dreiteiligen erfindungsgemäßen Ausführungsform die Konvexität (zweiteilige Prothese) bzw. der mittlere Gleitpartner (dreiteilige Prothese) bezüglich einer gedachten Horizontalen parallel oder nicht parallel ist. Bei einer nicht parallelen Ausführungsform stehen Ober- und Unterseite in einem Winkel in Bezug zur gedachten Horizontalen zueinander, wobei bei einem mittleren Gleitpartner der Winkel oben und unten gleich groß oder unterschiedlich sein kann. Die Konvexität(en) und korrespondierende(n) Konkavität(en) sind bei der zwei- und dreiteiligen Prothese in ihrer Oberflächengestaltung symmetrisch oder asymmetrisch. Durch die gewinkelte Konvexität bzw. den gewinkelten mittleren Gleitpartner sind ebenfalls Anpassungen an Asymmetrien des Wirbelkörperzwischenraumes möglich, in welchen die Prothese implantiert wird.

In einer bevorzugten Ausführungsform ist die transversal nach ventral verlaufende bogenförmige Krümmung der Konvexitäten von der Mitte nach lateral jeweils unterschiedlich. Die Konkavitäten der artikulierenden Gleitpartner weisen korrespondierende bogenförmige Krümmungen auf. Bei dieser asymmetrisch verlaufenden bogenförmigen Krümmung nach ventral ist alternativ zentral eine Unterbrechung der Konvexität vorgesehen, wobei in diesem Fall die jeweils korrespondierende Konkavität eine Unterbrechung in Form eines Steges aufweisen kann.

Die unterschiedlichen bogenförmigen Krümmungen bieten den Vorteil, dass dadurch Anpassungen an unterschiedliche Stellungen der Wirbelbogengelenke vorgenommen werden können. Dies ermöglicht eine optimale Integration der Facettenstellung der Wirbelbogengelenke in die asymmetrisch nach ventral gebogenen Konvexitäten bei der Rotation.

Zur sicheren Implantatverankerung im Zwischenwirbelraum dient eine randständige und/oder flächenhafte Verzahnung der Außenseiten von oberem und unterem Gleitpartner zur Verbindung mit einem oberen bzw. unteren Wirbelkörper. Die Außenseiten selbst sind plan oder konvex geformt und es ist möglich, die Verzahnung oder die gesamte Außenseite, auch ohne Verzahnung, bioaktiv oder stumpf zu beschichten. Um das Risiko einer Fraktur des Wirbelkörpers zu minimieren, ist eine Verankerung mit drei ventral angeordneten und zwei dorsal angeordneten Verankerungszähnen bevorteilt. Alternativ sind durchgehende laterale Zahnreihen vorn und hinten oder schräg verlaufende Zahnreihen bevorteilt, zur Prothesenimplantation von lateral und ventrolateral sowie zur besseren Führung des oberen und unteren Gleitpartners beim Einsetzen zwischen den Wirbelkörpern, da die Arbeitszange des Operateurs in die mittlere Lücke zwischen den Zahnreihen greifen kann oder auf Höhe der Zähne in Führungslöcher des oberen und unteren Gleitpartners.

Zur Vereinfachung der Im- oder Explantation der Bandscheibenendoprothese weisen oberer und/oder unterer Gleitpartner in einer weiteren Ausführungsform Vorsehungen für Instrumente auf. Diese Vorsehungen bestehen vorzugsweise aus Löchern oder Ausformungen, in die das jeweils benötigte Instrument des Operateurs eingreifen kann und so ein sicherer Halt des jeweiligen Gleitpartners ermöglicht wird.

Bei einer erfindungsgemäßen Bandscheibenendoprothese sind des weiteren als absolute Maße eine maximale Breite (Frontalansicht) von 14 bis 48 mm, eine maximale Tiefe (Sagittalschnitt) von 11 bis 35 mm und eine maximale Höhe von 4 bis 18 mm vorgesehenen. Diese Maße orientieren sich an den natürlichen Gegebenheiten der Lenden- und Halswirbelsäule und gewährleisten so, dass eine erfindungsgemäße Bandscheibenendoprothese der *in vivo* Situation möglichst nahe kommt.

Ferner sind bei einer erfindungsgemäßen Bandscheibenendoprothese ein oder mehrere röntgenkontrastgebende Markierungen vorgesehen, welche nicht röntgenkontrastgebende Teile der Prothese jeweils unterhalb ihrer Oberfläche enthalten. Dadurch ist es möglich, die Lage dieser Teile einer Bandscheibenendoprothese direkt nach der Implantation auf eine exakte Lage hin zu kontrollieren. Des weiteren ist es möglich, in definierten Zeitabständen durch Röntgen zu überprüfen, ob diese Teile der Prothese sich in ihrer Lage verändert haben bzw. immer noch exakt positioniert sind.

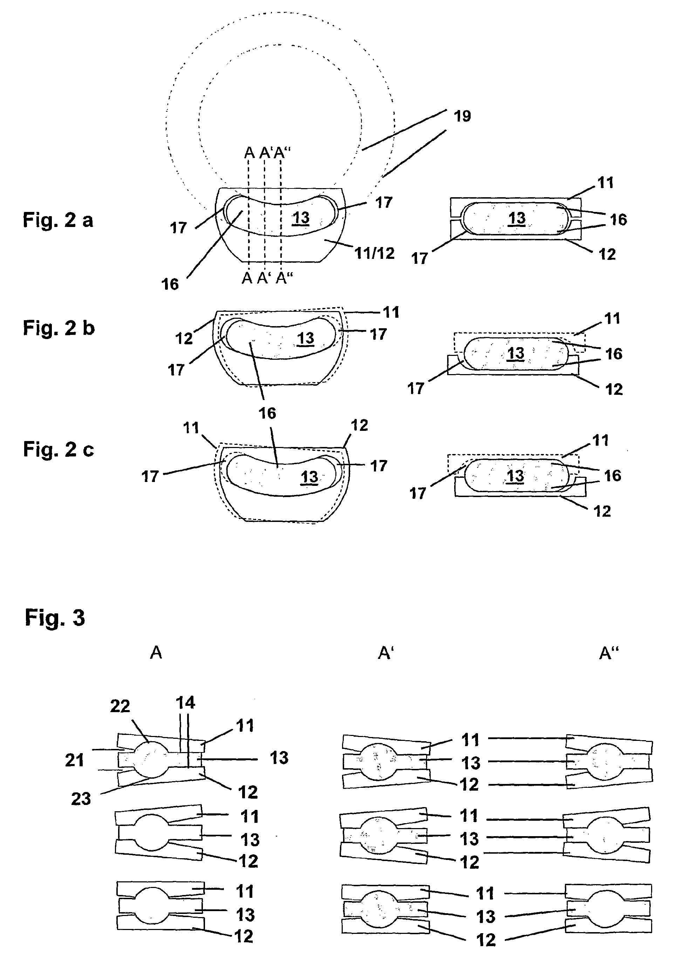

Weitere vorteilhafte Maßnahmen sind in den übrigen Unteransprüchen beschrieben; die Erfindung wird anhand von Ausführungsbeispielen und der nachfolgenden Figuren näher beschrieben; es zeigt:

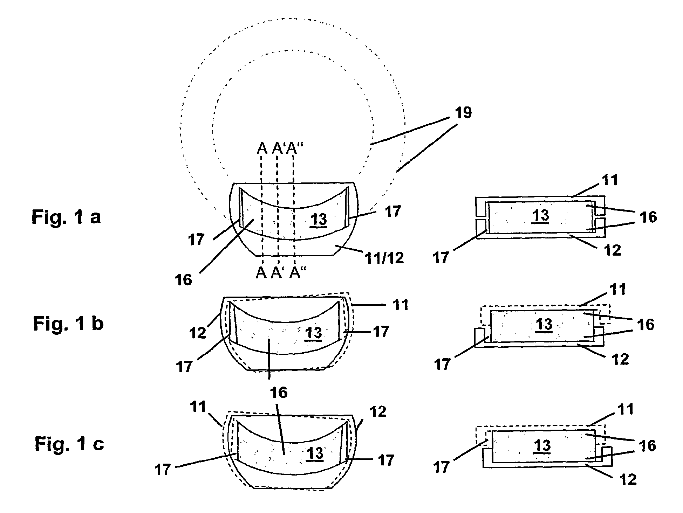

- **Figur 1a - c**: Schematische Darstellung einer Transversalansicht einer erfindungsgemäßen dreiteiligen Bandscheibenendoprothese mit nach ventral gekrümmter, artikulierender Konvexität und Konkavität, welche lateral nicht abgerundet sind (für die Lendenwirbelsäule):
a: schematische Darstellung ohne Rand des mittleren Gleitpartners bei mittiger Ausrichtung der Konvexität und deckungsgleicher Lage von oberem und unterem Gleitpartner, links mittlerer Transversalschnitt, rechts mittlerer Frontalschnitt
b: maximal im Gegenuhrzeigersinn gedrehter oberer Gleitpartner mit der Konkavität
c: maximal im Uhrzeigersinn gedrehter oberer Gleitpartner mit der Konkavität
- **Figur 2 a - c**: Schematische Darstellung einer Transversalansicht einer erfindungsgemäßen dreiteiligen Bandscheibenendoprothese mit nach ventral gekrümmter, jeweils lateral abgerundeter artikulierender Konvexität und Konkavität und nach dorsal versetzter Artikulationsfläche (für die Lendenwirbelsäule):
a: schematische Darstellung ohne Rand des mittleren Gleitpartners bei mittiger Ausrichtung der Konvexität und deckungsgleicher Lage von oberem und unterem Gleitpartner, links mittlerer Transversalschnitt, rechts mittlerer Frontalschnitt
b: maximal im Gegenuhrzeigersinn gedrehter oberer Gleitpartner mit der Konkavität
c: maximal im Uhrzeigersinn gedrehter oberer Gleitpartner mit der Konkavität
- **Figur 3**: Schematische Darstellung von Sagittalschnitten in den Ebenen A - A, A' - A' und A" - A" einer erfindungsgemäßen dreiteiligen Bandscheibenendoprothese (für die Lendenwirbelsäule) mit Oben: Lückenschluss der Gleitpartner ventral Mitte: Lückenschluss der Gleitpartner dorsal Unten: nicht geneigten
- **Figur 4 a - c**: Gleitpartnern Schematische Darstellung einer nicht unter die Ansprüche fallenden zweiteiligen Bandscheibenendoprothese mit Nullkrümmung
a: frontale Außenansicht und Frontalschnitt
b: Sagittalschnitte, links ohne, rechts mit Bewegung nach dorsal und ventral
c: Transversalansicht des oberen Gleitpartners mit Konkavität und nach dorsal versetzter Konkavität (für die Lendenwirbelsäule)
- **Figur 5 a, b**: Schematische Darstellung einer nicht unter die Ansprüche fallenden dreiteiligen Bandscheibenendoprothese mit Nullkrümmung
a: frontale Außenansicht und Frontalschnitt
b: Sagittalschnitte, links ohne, rechts mit Bewegung nach dorsal und ventral
- **Figur 6 a - c**: Schematische Darstellung verschiedener Formen des oberen und unteren Gleitpartners für die Lendenwirbelsäule
- **Figur 7 a, b**: Schematische Darstellungen der Anordnung von Verankerungszähnchen auf den Außenseiten des oberen und unteren Gleitpartners für die Lendenwirbelsäule

In den Figuren 1 a - c und 2 a - c ist jeweils links eine transversale Aufsicht auf die Gleitpartner 11, 12 einer erfindungsgemäßen dreiteiligen Bandscheibenendoprothese für die Lendenwirbelsäule mit gekrümmter, artikulierender Konvexität 16 und Konkavität 17 und des mittleren Gleitpartners 13 ohne Rand 14 abgebildet. Durch die gekrümmten Artikulationsflächen wird eine begrenzte Rotation von oberem und unterem Gleitpartner 11, 12 in Bezug auf eine gedachte Vertikalachse der Prothese ermöglicht. Konvexität 16 und Konkavität 17 sind in der dargestellten Ausführung nach dorsal versetzt.

Die ventrale und dorsale Krümmung der Konvexität 16 des mittleren Gleitpartners 13 ist von zwei Kreisen mit unterschiedlichen Radien 19, aber identischen Mittelpunkten abgeleitet. Die Krümmungsradien verlaufen in konstantem Abstand zueinander. Es ist erfindungsgemäß aber auch vorgesehen, dass die Kreisbögen nach lateral aufeinander zulaufen oder divergieren können. Im rechten Teil der Figuren 1 a - c und 2 a - c ist jeweils ein Frontalschnitt der im linken Teil gezeigten Prothese schematisch gezeigt. In den Figuren 1 a und 2 a ist die Lage der drei Schnittebenen A - A, A' - A' und A" - A" angegeben, auf die in Figur 3 jeweils eine sagittale Ansicht dargestellt ist.

In Figur 1 a ist sowohl im linken Teil als auch im rechten Teil der Figur gut zu erkennen, dass bei nicht gedrehten Gleitpartnern 11, 12, wenn also die Außenumfänge von oberem und unterem Gleitpartner 11, 12 in der Transversalansicht deckungsgleich übereinander liegen, rechts und links von der Konvexität 16 des mittleren Gleitpartners 13 die Konkavität 17 des oberen und unteren Gleitpartners 11, 12 etwas breiter ausgestaltet ist und somit "Spiel" für die Bewegung der Konvexität 16, des mittleren Gleitpartners 13 in der Konkavität 17 des oberen Gleitpartners 11 und unteren Gleitpartners 12 vorhanden ist. Die Konvexität 16 ist in Figur 1 a mittig in der Konkavität 17 ausgerichtet.

Figur 1b zeigt die Lage der Gleitpartner 11, 12, 13 einer erfindungsgemäßen Prothese zueinander, wenn der obere Gleitpartner 11, dessen Lage durch die gestrichelte Linie angegeben ist, gegen den Uhrzeigersinn bis zum Anschlag an den mittleren Gleitpartner 13 verdreht ist. Dabei ist rechts in Figur 1b zu erkennen, das sich auch die Position des mittleren Gleitpartners 13 in der Konkavität 17 ändert. Der mittlere Gleitpartner 13 wird bei der Drehung des oberen Gleitpartners bis zur rechten Außenseite der Konkavität 17 des oberen Gleitpartners 11 verschoben. Durch diesen Kontakt erfährt die Rotation ihre Begrenzung. In Figur 1c ist die Lage der Gleitpartner zueinander dargestellt, wenn der obere Gleitpartner 11, maximal im Uhrzeigersinn gegen den unteren Gleitpartner gedreht ist. Auch bei dieser Drehung bewegt sich der mittlere Gleitpartner 13 in der Konkavität 17 des unteren Gleitpartners 12. Im rechten Teil der Figuren 1b und c ist jeweils gut zu erkennen, dass sich die Außenumfänge von oberem und unterem Gleitpartner 11, 12 bei maximaler Drehung verschieben. Oberer und unterer Gleitpartner 11, 12 sind bezüglich ihrer seitlichen Außenflächen nicht mehr auf einer Linie. Bei einer implantierten erfindungsgemäßen Bandscheibenendoprothese stellt die Drehung der Gleitpartner 11, 12 zueinander eine Rotation in Bezug auf eine gedachte Vertikalachse der Prothese dar.

In den Figuren 1 a - c schließen die Oberflächen der Konvexität 16 und Konkavität 17 jeweils winklig ab. In den Figuren 2 a - c ist eine erfindungsgemäße Ausführungsform dargestellt, bei der die lateralen Enden von Konvexität 16 und Konkavität 17 jeweils abgerundet sind.

Figur 3 zeigt die sagittalen Querschnitte durch die in den Figuren 1 a - c und 2 a - c angegebenen Schnittebenen A - A, A' - A' und A" - A" für die Lendenwirbelsäule. Oben ist jeweils ein ventraler Lückenschluss der Ränder 14 der Gleitpartner 11, 12, 13 zu sehen. Dadurch vergrößert sich der Öffnungswinkel 21 auf der gegenüberliegenden Seite des konvex-konkaven Teils der Gleitflächen 22, 23. In der Mitte ist ein dorsaler Lückenschluss zu sehen und im unteren Teil jeweils nicht zueinander geneigte Gleitpartner 11, 12, 13. In den drei Schnittebenen ist gut zu erkennen, wie sich die Lage von oberer und unterer Gleitfläche 22, 23 in Richtung des mittigen Schnittes, also von Schnittebene A zu A", aufgrund der Krümmung in transversaler Ebene der Artikulationsflächen von dorsal nach ventral verlagert.

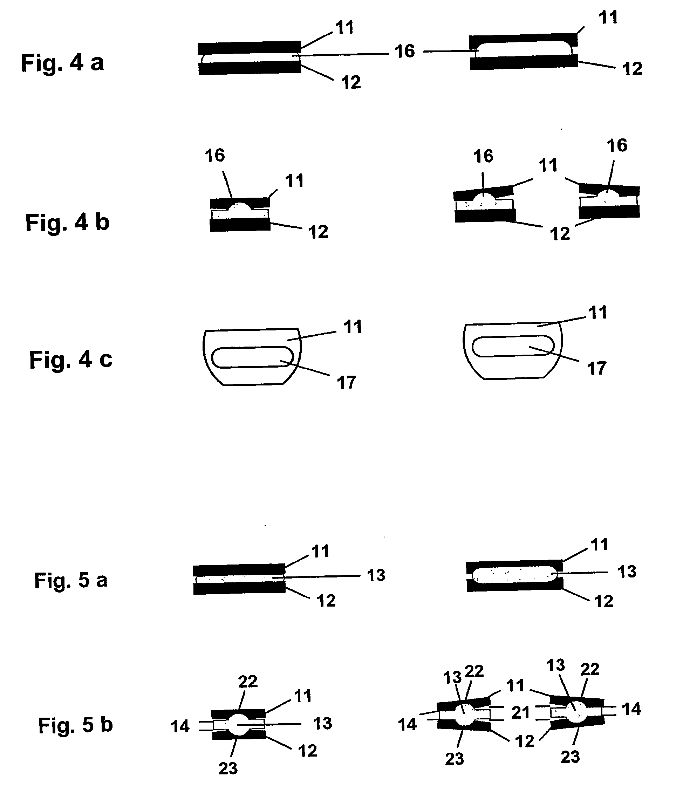

Figur 4 a - c zeigt eine schematische Darstellung einer zweiteiligen Bandscheibenendoprothese mit Nullkrümmung der Konvexität in Frontalansicht (Fig. 4a), Sagittalansicht (Fig. 4b) und Transversalansicht (Fig. 4c).

In Figur 4 a ist links eine frontale Außenansicht einer zweiteiligen Bandscheibenendoprothese mit oberem Gleitpartner 11 und unterem Gleitpartner 12 dargestellt. Der untere Gleitpartner 12 kann je nach Ausführungsform aus zwei Teilen bestehen, was durch die Farben grau und schwarz angedeutet ist. Dabei besteht der die Konvexität 16 aufweisende Teil (grau) je nach Ausführungsform aus einem anderen Material als der wirbelkörperseitige Teil des Gleitpartners 12 (schwarz). Bevorzugt sind oberer Gleitpartner 11 und unterer Gleitpartner 12 (schwarzer Teil) aus identischen Materialien und die Konvexität 16 besteht aus einem anderen Material. Es ist aber auch vorgesehen, dass die Gleitpartner 11, 12 und die Konvexität 16 aus gleichem Material bestehen.

Figur 4 a zeigt rechts einen Frontalschnitt durch eine funktionell zweiteilige Prothese mit oberem Gleitpartner 11, unterem Gleitpartner 12 und Konvexität 16, wobei der untere Gleitpartner aus zwei Teilen besteht.

In Figur 4b ist jeweils ein Sagittalschnitt durch eine zweiteilige Bandscheibenendoprothese zu sehen. Im linken Teil ist die Prothese in einer Position mit nicht geneigten Gleitpartnern 11, 12 zu sehen, wobei in 4 b rechts jeweils ein dorsaler und ventraler Lückenschluss dargestellt ist. In grau ist die Konvexität 16 dargestellt.

Figur 4c zeigt zwei verschiedene Aufsichten auf die Innenseiten eines oberen Gleitpartners 11 einer zweiteiligen Bandscheibenendoprothese. Eingezeichnet ist die konkave Ausnehmung 17, die eine zur einer Konvexität korrespondierende Form aufweist. Im rechten Teil der Abbildung ist die Konkavität nach dorsal (Lendenwirbelsäule) versetzt. Im Falle einer dreiteiligen Bandscheibenendoprothese zeigt Fig. 4c die Innenseite des oberen und unteren Gleitpartners mit der Konkavität, rechts bei dorsaler Versetzung (Lendenwirbelsäule).

In Figur 5 a sind rechts und links schematische Frontalansichten einer dreiteiligen Bandscheibenendoprothese mit Nullkrümmung, mit oberem Gleitpartner 11, unterem Gleitpartner 12 und mittlerem Gleitpartner 13 zu sehen. Figur 5 a zeigt links eine frontale Außenansicht einer funktionell dreiteiligen erfindungsgemäßen Bandscheibenendoprothese mit zylindrisch geformtem mittleren Gleitpartner 13, welcher an den Seiten abgerundet ist. In Figur 5 a rechts ist ein mittiger Frontalschnitt einer dreiteiligen Bandscheibenendoprothese dargestellt. In diesem Schnitt ist gut zu erkennen, dass die Höhe des mittleren Gleitpartner 13 von einer Seite zur anderen Seite konstant ist. Aus diesem Grund ist mit einer solchen zwei- und auch dreiteiligen Bandscheibenendoprothese kein Seitneigen der Gleitpartner zueinander möglich.

Figur 5b zeigt jeweils einen Sagittalschnitt einer dreiteiligen Prothese. Es ist jeweils der obere Gleitpartner 11, der untere Gleitpartner 12 sowie der dazwischen lokalisierte mittlere Gleitpartner 13 zu sehen. In Figur 5b sind der Außenumfang der oberen und unteren Konvexität Teil eines gemeinsamen Kreises. Es ist aber auch eine Ausführungsform möglich, bei welcher der mittlere Gleitpartner 13 abgeflacht ist und so die Konvexitäten nicht mehr Teil einer gemeinsamen Kreisbahn, sondern periphere Kreisausschnitte sind. Zudem können sich die Radien von oberer und unterer Konvexität unterscheiden. Die beiden zuletzt genannten Ausführungsformen sind in den Figuren nicht dargestellt.

In Figur 5b ist links die Prothese in einer Position mit nicht geneigten Gleitpartnern 11, 12, 13 zu sehen, wobei in Figur 5b rechts jeweils ein dorsaler und ventraler Lückenschluss dargestellt sind. Der Öffnungswinkel 21 hat sich in Figur 5b rechts jeweils entsprechend der geschlossenen Lücke auf der gegenüberliegenden Seite des konkav-konvexen Teils der Gleitflächen 22, 23 vergrößert. Es entsteht ein Lückenschluss zwischen den Rändern 14 bei endgradiger Neigung aller Gleitpartner 11, 12, 13, so dass eine optimale Druckverteilung gewährleistet ist.

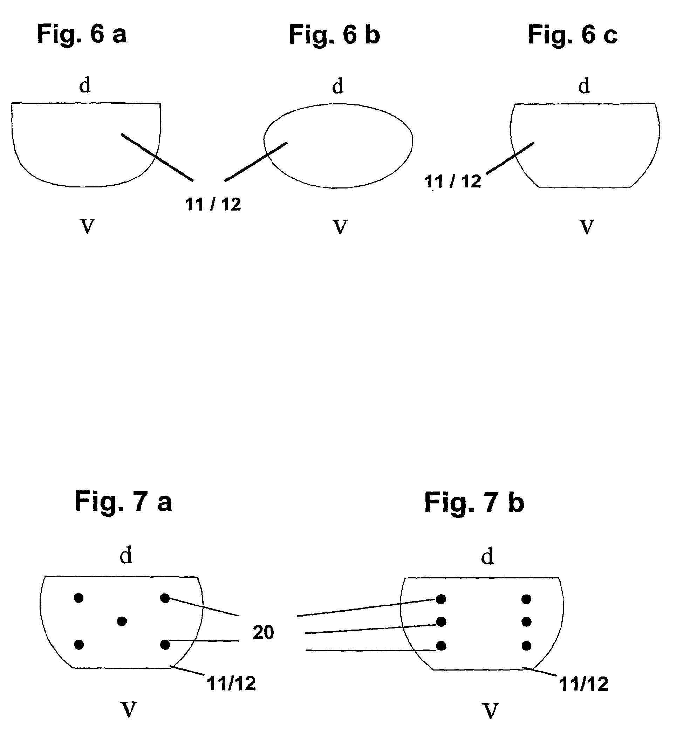

Die Figuren 6 a - c zeigen jeweils in einer Aufsicht auf oberen und unteren Gleitpartner 11, 12 schematisch alternative Gestaltungen der Form des Außenumfanges. Dabei ist mit den kleinen Buchstaben jeweils die Orientierung hinsichtlich der dorsoventralen Ausrichtung der Platten für die Lendenwirbelsäule angegeben (d = dorsal; v = ventral), die jedoch bei der Halswirbelsäule umgekehrt ist (v dann dorsal und d dann ventral).

In den Figuren 7 a und 7 b sind für die Lendenwirbelsäule alternative Anordnungen von Verankerungszähnchen 20 auf der Außenseite des oberen und unteren Gleitpartners 11, 12 dargestellt. Auch in dieser Zeichnung ist die Orientierung der Gleitpartner hinsichtlich der dorsoventralen Ausrichtung durch die kleinen Buchstaben kenntlich gemacht (d = dorsal; v = ventral). Dorsal ist in der Mitte jeweils kein Verankerungszähnchen 20 vorgesehen, da dies einerseits eine Schonung der Wirbelkörper bewirkt und andererseits die Implantation erleichtert. Die Anordnung der Verankerungszähnchen gestattet die Implantation der Bandscheibenendoprothese von ventral als auch von ventrolateral und lateral. Für die Halswirbelsäule gilt wieder die entgegengesetzte Orientierung, ebenfalls ohne mittleres dorsales Verankerungszähnchen 20.

Die in den Abbildungen dargestellten Ausführungsformen der erfindungsgemäßen Bandscheibenendoprothesen sowohl in einer zweiteiligen als auch in einer dreiteiligen Ausführung sind nur beispielhaft und nicht abschließend.

### Bezugszeichenliste

- 11: oberer Gleitpartner
- 12: unterer Gleitpartner
- 13: mittlerer Gleitpartner
- 14: Rand
- 16: Konvexität
- 17: Konkavität
- 19: Kreisumfang
- 20: Verankerungszähnchen
- 21: Öffnungswinkel
- 22: obere Gleitfläche
- 23: untere Gleitfläche

## Patentansprüche

1. Bandscheibenendoprothese für den vollständigen Ersatz der Bandscheibe im Lenden- und Halswirbelsäulenbereich, bestehend aus artikulierenden Gleitpartnern, wobei der obere Gleitpartner Mittel für eine feste Verbindung mit einem oberen Wirbelkörper und der untere Gleitpartner Mittel für eine feste Verbindung mit einem unteren Wirbelkörper aufweist und zwischen den Gleitpartnern eine Gleitfläche angeordnet ist, **dadurch gekennzeichnet, dass**
a) ein erster Gleitpartner (11, 12) derart ausgebildet ist, dass die zur Verbindung mit einem Wirbelkörper entgegengesetzte Seite eine konvexe Krümmung (16) aufweist, und die Geometrie der Konvexität (16) dadurch definiert ist, dass diese einem Abschnitt eines Zylinders entlang seiner Längsachse von rechts nach links lateral mit einer transversal nach ventral gerichteten bogenförmigen Krümmung entspricht, wobei die Konvexität (16) dorsal, ventral und beidseits lateral von einem Rand (14) abgeschlossen wird, und
b) ein zweiter Gleitpartner (11, 12) derart ausgebildet ist, dass die zur Verbindung mit einem Wirbelkörper entgegengesetzte Seite mit einer konkaven Artikulationsfläche (17) ausgebildet ist und die Geometrie der Konkavität (17) dadurch definiert ist, dass diese eine zur Konvexität (16) des ersten Gleitpartners (11, 12) korrespondierende Ausnehmung aufweist, und die Konkavität (17) dorsal, ventral und beidseits lateral von einem Rand (14) abgeschlossen wird, und
c) die Ränder (14) beider Gleitpartner (11, 12) einen sich nach außen öffnenden Winkel (Öffnungswinkel, 21) zueinander aufweisen, wobei
a. keine Neigung der Gleitpartner (11, 12) zueinander in lateraler Richtung möglich ist, und
b. die maximal mögliche Bewegung der Gleitpartner (11, 12) in dorsoventraler Richtung durch einen Lückenschluss der Ränder (14) der beiden Gleitpartner (11, 12) begrenzt wird, und
d) die Rotation der Gleitpartner zueinander begrenzt wird durch die Toleranz zwischen Konvexität (16) und Konkavität (17) rechts und links lateral der transversal gekrümmten zylindrischen Artikulationsfläche.

2. Bandscheibenendoprothese für den vollständigen Ersatz der Bandscheibe im Lenden- und Halswirbelsäulenbereich, bestehend aus artikulierenden Gleitpartnern, wobei der obere Gleitpartner Mittel für eine feste Verbindung mit einem oberen Wirbelkörper und der untere Gleitpartner Mittel für eine feste Verbindung mit einem unteren Wirbelkörper aufweist und zwischen oberem und unterem Gleitpartner ein weiterer, mittlerer Gleitpartner angeordnet ist, der mit den Innenseiten des oberen und unteren Gleitpartners derart korrespondiert, dass eine obere und eine untere Gleitfläche entstehen, **dadurch gekennzeichnet, dass**
a) der mittlere Gleitpartner (13) auf Ober- und Unterseite eine konvexe Krümmung (16) aufweist, und die Geometrie der Konvexitäten (16) dadurch definiert ist, dass diese einem Abschnitt eines Zylinders entlang seiner Längsachse von rechts nach links lateral mit einer transversal nach ventral gerichteten bogenförmigen Krümmung entspricht, und
b) oberer und unterer Gleitpartner (11, 12) mit einer konkaven inneren Artikulationsfläche (17) ausgebildet sind und die Geometrie der Konkavitäten (17) dadurch definiert ist, dass diese eine zur artikulierenden Konvexität (16) des mittleren Gleitpartners (13) korrespondierende Ausnehmung aufweist, und die Konkavitäten (17) auf Ober- und Unterseite jeweils dorsal, ventral und beidseits lateral von einem Rand (14) abgeschlossen werden, und
c) die Ränder (14) der artikulierenden Gleitpartner (11, 12,) einen sich nach außen öffnenden Winkel (Öffnungswinkel, 21) zueinander aufweisen, wobei
a. keine Neigung der Gleitpartner (11, 12) zueinander in lateraler Richtung möglich ist, und
b. die maximal mögliche Bewegung der Gleitpartner (11,12) in dorsoventraler Richtung durch einen Lückenschluss der Ränder (14) der Gleitpartner (11, 12) begrenzt wird, und
d) die Rotation der Gleitpartner zueinander begrenzt wird durch die Toleranz zwischen Konvexitäten und Konkavitäten rechts und links lateral der transversal gekrümmten zylindrischen Artikulationsflächen.

3. Bandscheibenendoprothese nach Anspruch 2, wobei sich die Konvexitäten (16) des mittleren Gleitpartners (13) jeweils über die gesamte Ober- und Unterseite erstrecken oder die Konvexitäten von einem Rand (14) umgeben sind.

4. Bandscheibenendoprothese nach wenigstens einem der Ansprüche 1 bis 3, wobei nach ventral eine größere Neigung der Gleitpartner (11, 12 und 13) zueinander möglich ist als nach dorsal.

5. Bandscheibenendoprothese nach wenigstens einem der Ansprüche 1 bis 4, wobei eine zylindrische Konvexität (16) entlang ihrer Längsachse sich einseitig konisch verringernde Radien aufweist und der Rand (14) an die Verringerung der Radien angepasst ist.

6. Bandscheibenendoprothese nach wenigstens einem der der Ansprüche 1 bis 5, wobei ventraler und dorsaler Krümmungsradius von bogenförmig gekrümmten Konvexitäten (16) und korrespondierenden Konkavitäten (17) gleich sind oder sich voneinander unterscheiden.

7. Bandscheibenendoprothese nach wenigstens einem der Ansprüche 1 bis 6, wobei die maximal mögliche Bewegung der Gleitpartner (11, 12, und 13)
a) bei der dorsoventralen Neigung der Gleitpartner (11, 12, und 13) zueinander durch den Krümmungsradius sowie die Höhe der Konvexität (16) und Konkavität (17) und des jeweiligen Randes (14), der eine Artikulationsfläche ventral und dorsal umgibt, und
b) den Öffnungswinkel (21) zwischen den Rändern (14) benachbarter Gleitpartner (11, 12, und 13), wobei dieser durch schräg und/oder waagerecht verlaufende Ränder (14) der benachbarten Gleitpartner (11, 12, und 13) gebildet wird, wobei es bei endgradigem Kontakt der Gleitpartner (11, 12 und 13) zu einem Lückenschluss kommt, und
c) die Rotation der Gleitpartner in Bezug auf eine gedachte Vertikalachse, durch die bogenförmige(n) Krümmung(en) und das Ausmaß der bogenförmigen Krümmung(en) der zylindrischen Konvexität(en) und der korrespondierenden Konkavität(en) und die Toleranz zwischen Konvexität und Konkavität jeweils rechts und links lateral am Ende der zylindrischen Artikulationsflächen,
festgelegt ist.

8. Bandscheibenendoprothese nach wenigstens einem der Ansprüche 1 bis 7, wobei die Gleitpartner (11, 12 und 13) einstückig ausgebildet sind.

9. Bandscheibenendoprothese nach wenigstens einem der Ansprüche 1 bis 7, wobei wenigstens ein Gleitpartner (11, 12 und 13) aus wenigstens zwei fest oder fest, aber reversibel miteinander verbundenen Teilen besteht, wobei entweder die Konvexität(en) (16) und/oder Konkavität(en) (17) den Teil ausmachen, der fest oder fest, aber reversibel mit dem jeweiligen Gleitpartner (11, 12 und 13) verbunden ist, oder Konvexität(en) (16) und/oder Konkavität(en) (17) an der Basis geeignete Mittel für eine feste oder feste, aber reversible Verbindung mit dem jeweiligen Gleitpartner aufweisen.

10. Bandscheibenendoprothese nach wenigstens einem der vorhergehenden Ansprüche, wobei die Gleitpartner (11, 12 und 13) und Konvexität(en) (16) und/oder Konkavität(en) (17) sowie der umgebende Rand (14) aus gleichen oder verschiedenen Materialien bestehen.

11. Bandscheibenendoprothese nach wenigstens einem der vorhergehenden Ansprüche, wobei die Oberflächen der Gleitpartner (11, 12 und 13) und/oder miteinander verbundenen Teile gleich oder verschieden beschichtet sind.

12. Bandscheibeztendoprothese nach Anspruch 9-11, wobei die feste oder feste, aber reversible Verbindung zwischen Gleitpartner(n) (11, 12 und 13) und Konvexität(en) (16) oder Konkavität(en) (17) durch eine Nut/Feder-Verbindung, eine Führungsschiene und korrespondierende Ausnehmung, einen Schnappmechanismus, Kleben oder Verschrauben hergestellt ist.

13. Bandscheibenendoprothese nach wenigstens einem der Ansprüche 2 bis 12, wobei oberer und unterer Gleitpartner (11, 12) aus dem gleichen Material bestehen oder gleich beschichtet sind und der mittlere Gleitpartner (13) aus einem anderen Material gefertigt ist oder anders beschichtet ist.

14. Bandscheibenendoprothese nach wenigstens einem der vorhergehenden Ansprüche, wobei die maximale Höhe einer Konvexität (16) geringer ist als ein Halbzylinder und/oder die Höhe des Randes (14) verringert ist, wobei bei einer dreiteiligen Bandscheibenendoprothese die Höhe der Konvexitäten (16) auf Ober- und Unterseite gleich oder unterschiedlich ist.

15. Bandscheibenendoprothese nach Anspruch 2 oder wenigstens einem darauf rückbezogenen vorhergehenden Anspruch, wobei die Krümmungsradien der Konvexitäten (16) auf Ober- und Unterseite des mittleren Gleitpartners (13) sowie die korrespondierenden Konkavitäten (17) auf oberem und unterem Gleitpartner (11, 12) identisch oder unterschiedlich sind.

16. Bandscheibenendoprothese nach wenigstens einem der vorhergehenden Ansprüche, wobei der maximale Öffnungswinkel bei einseitigem Lückenschluss der Gleitpartner (11, 12 und 13) aus der Extension oder Flexion zwischen 6 und 10 Grad beträgt, mit einer Toleranz von zusätzlich je 3 Grad in jede Richtung.

17. Bandscheibenendoprothese nach wenigstens einem der vorhergehenden Ansprüche, wobei die Rotation um eine gedachte zentrale Vertikalachse bei nach ventral gerichteter bogenförmiger Krümmung von Konvexität(en) (16) und Konkavität(en) (17) zwischen den benachbarten Gleitpartnern (11, 12 und 13) abgebremst wird und eine begrenzte Rotationsbewegung der Gleitpartner (11, 12 und 13) im Bezug auf eine gedachte zentrale Vertikalachse um bis zu 3 Grad für die Lendenwirbelsäule und um bis zu 6 Grad für die Halswirbelsäule nach jeder Seite möglich ist, mit einer Toleranz von zusätzlich je 2 Grad nach jeder Seite.

18. Bandscheibenendoprothese nach wenigstens einem der vorhergehenden Ansprüche, wobei Konvexität(en) (16) und zugehörige korrespondierende Konkavität(en) (17) um bis zu 4 mm vom mittigen Frontalschnitt nach dorsal versetzt sind.

19. Bandscheibenendoprothese nach wenigstens einem der vorhergehenden Ansprüche, wobei die Ränder (14) der Gleitpartner (11, 12 und 13) ventral, lateral und dorsal außen rechtwinklig, anderweitig winklig, gekrümmt oder kombiniert gerade, gekrümmt und/oder winklig abgeschlossen sind.

20. Bandscheibenendoprothese nach Anspruch 2 oder wenigstens einem darauf rückbezogenen vorhergehenden Anspruch, wobei die Konvexitäten des mittleren Gleitpartners (13) im äußeren, beidseits lateralen Bereich symmetrisch oder asymmetrisch, rechtwinklig, anderweitig winklig, halbkugelförmig, abgerundet oder abgeflacht ausgestaltet sind.

21. Bandscheibenendoprothese nach Anspruch 2 oder wenigstens einem darauf rückbezogenen vorhergehenden Anspruch, wobei zur Sicherung des mittleren Gleitpartners (13) gegen ein Herausgleiten aus der Prothese bei Lückenschluss aller drei Gleitpartner (11, 12, 13) ein Anschlag Teil des Randes (14) des mittleren Gleitpartners (13) ist, der außerhalb des oberen und/oder unteren Gleitpartners (11, 12) angeordnet ist, wobei der Anschlag wenigstens auf Ober- oder Unterseite höher ist als der Rand (14) des mittleren Gleitpartners (13).

22. Bandscheibenendoprothese nach Anspruch 2 oder wenigstens einen darauf rückbezogenen vorhergehenden Anspruch, wobei zur zusätzlichen Sicherung des mittleren Gleitpartners (13) gegen ein Herausgleiten aus der Prothese beim Lückenschluss aller drei Gleitpartner (11, 12, 13), ein Anschlag Teil des Randes (14) des mittleren Gleitpartners (13) ist, der auf Ober- und/oder Unterseite höher als der Rand (14) des mittleren Gleitpartners (13) ist und innerhalb einer Nut vom Randbereich des oberen und/oder unteren Gleitpartners (11, 12) mit dem notwendigen Spiel für die maximale Gleitbewegung der Gleitpartner (11, 12, 13) geführt wird.

23. Bandscheibenendoprothese nach Anspruch 2 oder wenigstens einem darauf rückbezogenen vorhergehenden Anspruch, wobei zur zusätzlichen Sicherung eines mittleren Gleitpartners (13) mit Rand (14) gegen ein Herausgleiten aus der Prothese, beim Lückenschluss aller drei Gleitpartner (11, 12, 13), der Rand (14) des mittleren Gleitpartners (13) ab dem Übergangsbereich der Konvexität nach peripher teilweise oder insgesamt kontinuierlich höher wird und der Rand des oberen und/oder unteren Gleitpartners (11,12) im gleichen Maße flacher wird.

24. Bandscheibenendoprothese nach Anspruch 2 oder wenigstens einem darauf rückbezogenen vorhergehenden Anspruch, wobei zur zusätzlichen Sicherung eines mittleren Gleitpartners (13) gegen ein Herausgleiten aus der Prothese, beim Lückenschluss der drei Gleitpartner (11, 12, 13) die äußeren Kanten von oberem und/oder unterem Gleitpartner (11, 12) vollständig oder partiell hakenförmig, rechtwinklig, anderweitig winklig, gekrümmt oder in Kombinationen davon in Richtung des anderen äußeren Gleitpartners ausgestaltet sind.

25. Bandscheibenendoprothese nach wenigstens einem der vorhergehenden Ansprüche, wobei Fläche und Form des Außenumfanges des oberen und unteren Gleitpartners (11, 12) gleich oder ungleich sind und so an die jeweilige Größe des Wirbelkörpers, mit dem sie verbunden werden, angepasst sind.

26. Bandscheibenendoprothese nach wenigstens einem der vorhergehenden Ansprüche, wobei oberer und/oder unterer Gleitpartner (11, 12) im Frontal- und/oder Sagittalschnitt derart ausgebildet sind, dass die Außen- und Innenseite von oberem und/oder unterem Gleitpartner (11, 12) parallel oder nicht parallel zueinander verlaufen.

27. Bandscheibenendoprothese nach wenigstens einem der vorhergehenden Ansprüche, wobei Konvexität einer zweiteiligen Prothese (11, 12) und Ober- und Unterseite des mittleren Gleitpartners (13) einer dreiteiligen Prothese in Bezug zu einer Horizontalen parallel oder nicht parallel sind, und dann zueinander in einem definierten Winkel stehen, wobei die Konvexität(en) symmetrisch oder asymmetrisch sind.

28. Bandscheibenendoprothese nach wenigstens einem der vorhergehenden Ansprüche, wobei die transversal nach ventral bogenförmige Krümmung der Konvexität (16) von zentral nach beidseits lateral symmetrisch oder asymmetrisch ausgestaltet ist.

29. Bandscheibenendoprotlaese nach Anspruch 28, wobei die Konvexität (16) in der Mitte eine Unterbrechung und die korrespondierende Konkavität (17) eine Unterbrechung in Form eines Steges aufweist.

30. Bandscheibenendoprothese nach wenigstens einem der vorhergehenden Ansprüche, wobei der obere und untere Gleitpartnern (11, 12) auf der Außenseite plan oder konvex und bioaktiv beschichtet oder stumpf sind und zu deren Primärverankerung mit den Wibelkörpern Reihen von Verankerungszähnchen (20) aufweisen, welche entweder von dorsal nach ventral gerade oder schräg angeordnet sind oder in lateraler Ausrichtung angeordnet sind, wobei sich in der dorsalen Reihe nur an den Seiten Verankerungszähnchen (20) befinden.

31. Bandscheibenendoprothese nach wenigstens einem der vorhergehenden Ansprüche, wobei der obere und/oder untere Gleitpartner (11, 12) Mittel zum Eingreifen eines Instrumentes zur Im- oder Explantation aufweisen.

32. Bandscheibenendoprothese nach wenigstens einem der vorhergehenden Ansprüche, wobei diese eine maximale Breite (Frontalansicht) von 14 bis 48 mm, eine maximale Tiefe (Sagittalschnitt) von 11 bis 35 mm und eine maximale Höhe von 4 bis 18 mm aufweist.

33. Bandscheibenendoprothese nach wenigstens einem der vorhergehenden Ansprüche, geeignet für die Implantation in die Lendenwirbelsäule, wobei sich der Außenumfang des oberen und unteren Gleitpartners (11, 12) in transversaler Ansicht nach ventral verjüngt.

34. Bandscheibenendoprothese nach wenigstens einem der Ansprüche 1 bis 32 geeignet für die Implantation in die Halsrvirbelsäule, wobei sich der Außenumfang des oberen und unteren Gleitpartners (11, 12) in transversaler Ansicht nach dorsal verjüngt.

35. Bandscheibenendoprothese nach Anspruch 33 oder 34, wobei die Verjüngung des Außenumfangs des oberen und unteren Gleitpartners (11, 12) lateral jeweils als identische Krümmungen oder asymmetrisch ausgebildet sind.

36. Bandscheibenendoprothese nach wenigstens einem der vorhergehenden Ansprüche, wobei die Prothese (11, 12, und 13) jeweils unterhalb der Oberfläche ein oder mehrere röntgenkontrastgebende Markierungen enthalten.

## Claims

1. Intervertebral disc prosthesis for the total replacement of the intervertebral disc within the lumbar and cervical spine, comprising articulating sliding partners, wherein an upper sliding partner has means for a firm assembly to an upper vertebral body and the lower sliding partner has means for a firm assembly to a lower vertebral body and an in-between the sliding partners located sliding area, **characterized by**
a) a first sliding partner (11, 12) is constructed in such a manner, that a side opposite of a side for assembly with a vertebral body has a convex curvature (16), and the geometry of the convexity (16) is defined in that the geometry/convexity corresponds to a segment of a cylinder along its longitudinal axis from right to left lateral with a transversal ventrally directed arched curvature, wherein the convexity (16) is surrounded dorsally, ventrally and to both lateral sides by an edge (14), and
b) a second sliding partner (11, 12) constructed in such a manner, that the opposite side of the side for the assembly with a vertebral body is built with a concave articulation area (17) and the geometry of the concavity (17) is defined by the fact that it has a recess corresponding to the convexity (16) of the first sliding partner (11, 12), and the concavity (17) is surrounded dorsally, ventrally and to both lateral sides by an edge (14), and
c) the edges (14) of both sliding partners (11, 12) have an outwardly opening angle (aperture angle, 21) towards each other, wherein
a. no inclination of the sliding partners (11,12) towards each other in lateral direction being possible, and
b. the maximally possible motion of the sliding partners (11, 12) in dorsoventral direction is limited by a gap-closure of the edges (14) of the two sliding partners (11, 12), and
d) the rotation of the sliding partners (11,12) one with respect to the other is limited by the tolerance between convexity (16) and concavity (17) right and left laterally of the transversally curved cylindrical articulation areas.

2. Intervertebral disc prosthesis for the total replacement of the intervertebral disc within the lumbar and cervical spine, comprising articulating sliding partners, wherein the upper sliding partner has means for a firm assembly to an upper vertebral body and the lower sliding partner has means for a firm assembly to a lower vertebral body and wherein, between the upper and lower sliding partner, a further, middle sliding partner is located, corresponding in such a way to the inner surfaces of the upper and lower sliding partner that an upper and a lower sliding surface is formed, **characterized by**
a) the middle sliding partner (13) having on the upper and lower surfaces a convex curvature (16), and a geometry of the convexities (16) is defined by the fact that it corresponds to a segment of a cylinder along its longitudinal axis from right to left lateral with a transversal ventrally directed arched curvature, and
b) upper and lower sliding partner (11, 12) have a concave inner articulation area (17), and a geometry of the concavities (17) being defined by a corresponding recess to the articulating convexity (16) of the middle sliding partner (13), and the concavities (17) of the upper and lower side being surrounded dorsally, ventrally and laterally at both sides by an edge (14), and
c) the edges (14) of the articulation sliding partners (11, 12) having an outwardly opening angle (aperture angle, 21) towards each other, wherein
a. no inclination of the sliding partners (11, 12) towards each other in lateral direction is possible, and
b. the maximal possible motion of the sliding partners (11, 12) in dorsoventral direction is limited by a gap-closure of the edges (14) of the sliding partners (11, 12), and
d) the rotation of the sliding partners one with respect to the other is limited by the tolerance between the convexities and concavities right and left laterally of the transversally curved cylindrical articulation areas.

3. Intervertebral disc prosthesis according to claim 2, where each of the convexities (16) of the middle sliding partner (13) extend across the complete upper and lower side or the convexities are surrounded by an edge (14).

4. Intervertebral disc prosthesis, according to at least one of the claims 1 to 3, wherein a greater inclination of the sliding partners (11, 12, 13) towards each other in ventral direction than in dorsal direction is possible.

5. Intervertebral disc prosthesis according top at least one of the claims 1 to 4, wherein a cylindrical convexity (16) along a longitudinal axis has on one side conically tapering off radii and the edge (14) is adapted to the reduction of the radii.

6. Intervertebral disc prosthesis according to at least one of the claims 1 to 5, wherein ventral and dorsal radii of curvature of the convexities (16) that are curved like an arche and corresponding concavities (17) are equal or differ.

7. Intervertebral disc prosthesis according to at least one of the claims 1 to 6, wherein the maximal possible motion of the sliding partners (11, 12, 13) is defined by
a) a dorsoventral inclination of the sliding partners (11, 12, 13) towards each other by a radius of curvature as well as the height of the convexity (16) and concavity (17) and the respective edge (14) surrounding an articulation area ventrally and dorsally, and
b) the aperture angle (21) between the edges (14) of neighboring sliding partners (11, 12, 13), which is formed by the edges (14) running at an incline and/or horizontally of the neighboring sliding partners (11, 12, 13) wherein during terminal contact of the sliding partners (11, 12, 13) a gap-closure occurs, and
c) the rotation of the sliding partners, with respect to a fictitious vertical axis, by the arched curvature(s) and the extent of the arched curvature(s) of the cylindrical convexity(ies) and the corresponding concavity(ies) and a tolerance between convexity and concavity to the right and left laterally at the end of the cylindrical articulation areas respectively.

8. Intervertebral disc prosthesis according to at least on of the claims 1 to 7, wherein the sliding partners (11, 12, 13) are constructed in one piece.

9. Intervertebral disc prosthesis according to at least one of the claims 1 to 7, wherein at least one sliding partner (11, 12, 13) comprises of at least two permanent or permanently, but reversibly attached parts, with either the convexity(ies) (16) and/or the concavity(ties) (17) being the part that is permanent or permanently, but reversibly attached to the corresponding sliding partner (11, 12 and 13), or the convexity(ies) (16) and/or concavity(ies) (17) having suitable means for a permanent or permanent, but reversible assembly with the respective sliding partner on their base.

10. Intervertebral disc prosthesis according to at least one of the preceding claims, wherein the sliding partners (11, 12 and 13) and the convexity(ies) (16) and/or concavity(ies) (17) as well as the surrounding edge (14) consist of the same or different materials.

11. Intervertebral disc prosthesis according to at least one of the preceding claims, wherein the surfaces of the sliding partners (11, 12 and 13) and/or with each other connected parts are coated equally or differently.

12. Intervertebral disc prosthesis according to claims 9 to 11, wherein a permanent or permanent, but reversible assembly between sliding partners (11, 12 and 13) and convexity(ies) (16) or concavity(ies) (17) is provided by a groove/spring assembly, a guide tail and corresponding recess, a snap mechanism, gluing or screwing.

13. Intervertebral disc prosthesis according to at least one of the claims 2 to 12, wherein upper and lower sliding partner (11, 12) consist of the same material or are equally coated and the middle sliding partner (13) is made of a different material or is differently coated.

14. Intervertebral disc prosthesis according to at least one of the preceding claims wherein the maximal height of a convexity (16) is less than a semicylinder and/or the height of the edge (14) is reduced, wherein, in case of a three part intervertebral disc prosthesis, the height of the convexities (16) is the same or differs on upper and lower side.

15. Intervertebral disc prosthesis according to claim 2 or at least one preceding claim referring to it, wherein the radii of curvature of the convexities (16) on the upper and lower side of the middle sliding partner (13) as well as the corresponding concavities (17) on the upper and lower sliding partner (11, 12) are identical or different.

16. Intervertebral disc prosthesis according to at least one of the preceding claims, wherein the maximal aperture angle during one-sided gap-closure of the sliding partners (11, 12 and 13) during extension or flexion is between 6 and 10 degrees with a tolerance of an additional 3 degrees in every direction.

17. Intervertebral disc prosthesis according to at least one of the preceding claims, wherein the rotation around a fictitious central vertical axis between the neighboring sliding partners (11, 12, 13) is slowed down for ventrally directed arched curvature of the convexity(ies) (16) and concavity(ies) (17) and a limited rotation motion between the sliding partners (11, 12, 13) with respect to a fictitious central vertical axis of up to 3 degrees for the lumbar spine and up to 6 degrees for the cervical spine to every side is possible, with a tolerance of an additional 2 degrees to every side.

18. Intervertebral disc prosthesis according to at least one of the preceding claims, wherein the convexity(ies) (16) and the respective corresponding concavity(ies) (17) are dorsally displaced up to 4 mm away from a median section.

19. Intervertebral disc prosthesis according to at least one of the preceding claims, wherein the edges (14) of the sliding partners (11, 12 and 13) end ventrally, laterally and dorsally outwardly perpendicular, otherwise angled, curved or a combination of straight, curved, and /or angular.

20. Intervertebral disc prosthesis according to claim 2 or at least one claim referring to it, wherein the convexities of the middle sliding partner (13) are constructed asymmetrically, perpendicularly, otherwise angled, semispherically , rounded off or flattened, in both outer lateral regions.

21. Intervertebral disc prosthesis according to claim 2 or at least one claim referring to it, wherein as an additional safeguard for a middle sliding partner (13) against a slip-out out of the prosthesis during gap closure of all three sliding partners (11, 12, 13) a stop is part of the edge (14) of the middle sliding partner (13), that is located outside the upper and/or lower sliding partner (11, 12), and the stop on at least the upper or the lower side is higher than the edge (14) of the middle sliding partner (13).

22. Intervertebral disc prosthesis according to claim 2 or at least one of the claims referring to it, wherein as an additional safeguard for the middle sliding partner (13) against a slip-out out of the prosthesis during a gap-closure of all three sliding partners (11, 12, 13) a stop is part of the edge (14) of the middle sliding partner (13), which is higher on the upper and/or the lower side than the edge (14) of the middle sliding partner (13) and is guided within a groove in an edge area of the upper and/or lower sliding partner (11, 12) with clearance necessary for the maximal sliding motions of the sliding partners (11, 12, 13).

23. Intervertebral disc prosthesis according to claim 2 or at least one claim referring to it, wherein as an additional safeguard for the middle sliding partner (13) with edge (14) against a slip-out out of the prosthesis during a gap-closure of all three sliding partners (11, 12, 13) the edge (14) of the middle sliding partner (13) partly or totally increases in height from a transition area of the convexity to a periphery and the edge of the upper and/or lower sliding partner (11,12) levelling off by the same amount.

24. Intervertebral disc prosthesis according to claims 2 or at least one claim referring to it, wherein as an additional safeguard for the middle sliding partner (13) with edge (14) against a slip-out out of the prosthesis during a gap-closure of the three sliding partners (11, 12, 13), the most outward edges of the upper and/or lower sliding partner (11, 12) are completely or partially hook-shaped, perpendicular, otherwise angular, curved or a combination thereof in the direction of the other external sliding partner.

25. Intervertebral disc prosthesis according to at least one of the preceding claims, wherein surface and shape of the outer circumference of the upper and lower sliding partner (11, 12) are equal or unequal and can thereby be adapted to the corresponding size of the vertebral body with which they are to be assembled.

26. Intervertebral disc prosthesis according to at least one of the preceding claims, wherein the upper and/or lower sliding partner (11, 12) are designed in such a way that in the frontal and/or sagittal view the outside and inside of the upper and/or lower sliding partners (11, 12) run parallel or non-parallel relative to each other.

27. Intervertebral disc prosthesis according to at least one of the preceding claims, wherein the convexity of a two part prosthesis (11, 12) and upper and lower side of the middle sliding partner (13) of a three part prosthesis are parallel or non-parallel with respect to a horizontal and then stand towards each other in a defined angle, with the convexities being symmetrical or asymmetrical.

28. Intervertebral disc prosthesis according to at least one of the preceding claims, wherein the transversally ventrally arched curvature of the convexity (16) is constructed symmetrically or asymmetrically from central to both lateral sides.

29. Intervertebral disc prosthesis according to claim 28, wherein the convexity (16) has a discontinuation in the middle and the corresponding concavity (17) has a discontinuation in the form of a bar.

30. Intervertebral disc prosthesis according to at least one of the preceding claims, wherein the upper and lower sliding partner (11, 12) are plane or convex and coated bio-actively or blunt on the outer surface and have for their primary anchorage with the vertebral bodies special anchoring teeth (20), that are either arranged from dorsal to ventral straight or at an incline in lateral direction, wherein a respective dorsal row has only laterally arranged anchoring teeth (20).

31. Intervertebral disc prosthesis according to at least one of the preceding claims, wherein the upper and/or lower sliding partner (11, 12) has means for an instrument to grip for implantation or explantation.

32. Intervertebral disc prosthesis according to at least one of the preceding claims, having a maximal breadth (frontal view) of 14 to 48 mm, a maximal depth (sagittal view) of 11 to 35 mm and a maximal height of 4 to 18 mm.

33. Intervertebral disc prosthesis according to at least one of the preceding claims, suitable for the implantation into the lumbar spine, and wherein an outer circumference of the upper and lower sliding partner (11, 12) tapers off ventrally in the transversal view.

34. Intervertebral disc prosthesis according to at least one of the claims 1 to 32, suitable for the implantation into a cervical spine, and wherein the outer circumference of the upper and lower sliding partner (11, 12) tapers off dorsally in the transversal view.

35. Intervertebral disc prosthesis according to claims 33 or 34, wherein the tapering off of the outer circumference of the upper and lower sliding partners (11, 12), has each laterally identical curvatures or are asymmetric.

36. Intervertebral disc prosthesis according to at least one of the preceding claims, wherein the prosthesis (11, 12 and 13) comprises under each surface one or more radiolucent tags.

## Revendications

1. Endoprothèse de disque intervertébral pour le remplacement intégral de disque intervertébral dans la zone des vertèbres lombaires et des vertèbres cervicales, composée d'éléments glissants s'articulant, l'élément glissant supérieur comportant des moyens pour une fixation fixe avec un corps vertébral supérieur et l'élément glissant inférieur comportant des moyens pour une fixation fixe avec un corps vertébral inférieur et dans laquelle une surface de glissement est disposée entre les éléments glissants, **caractérisée en ce que**
a) un premier élément glissant (11, 12) est conçu de sorte à ce qu'il comporte une courbure convexe (16) pour la liaison à un côté opposé au corps vertébral, et la géométrie de la convexité (16) est définie **en ce que** celle-ci correspond à une section d'un cylindre le long de son axe longitudinal de droite à gauche latéralement avec une courbure en forme d'arc orientée dans le sens transversal-ventral, la convexité (16) étant fermée dorsalement, ventralement et latéralement des deux côtés par une bordure (14), et
b) un second élément glissant (11, 12) conçu de sorte qu'il est conçu pour une liaison avec le côté opposé d'un corps vertébral avec une surface d'articulation concave (17) et la géométrie de la concavité (17) est définie **en ce que** celle-ci comporte un évidement correspondant à la convexité (16) du premier élément glissant (11, 12), et la convexité (17) étant fermée dorsalement, ventralement et latéralement des deux côtés par une bordure (14), et
c) les bordures (14) des deux éléments glissants (11, 12) comportent un angle (angle d'ouverture, 21) s'ouvrant vers l'extérieur l'un par rapport à l'autre,
a. aucune inclinaison des éléments glissants (11, 12) n'étant possible dans la direction latérale et
b. le mouvement maximal possible des éléments glissants (11, 12) étant limité dans le sens dorsoventral par une fermeture interstitielle des bordures (14) des deux éléments glissants (11, 12) et
d) la rotation des éléments glissants l'un par rapport à l'autre est limitée par la tolérance entre la convexité (16) et la concavité (17) à droite et à gauche latéralement de la surface d'articulation cylindrique courbée transversalement.

2. Endoprothèse de disque intervertébral pour un remplacement intégral de disque intervertébral dans la zone des vertèbres lombaires et des vertèbres cervicales, composée d'éléments glissants s'articulant, l'élément glissant supérieur comportant un moyen pour une fixation fixe avec un corps vertébral supérieur et l'élément glissant inférieur comportant un moyen pour une fixation fixe avec un corps vertébral inférieur, un autre élément glissant central étant disposé entre l'élément glissant supérieur et inférieur, l'élément glissant central correspondant aux côtés intérieurs de l'élément glissant supérieur et inférieur de sorte à former une surface de glissement supérieure et inférieure, **caractérisée en ce que**
a) l'élément glissant central (13) comporte une courbure convexe (16) sur le côté supérieur et inférieur, et la géométrie de la convexité (16) est définie **en ce que** celle-ci correspond à une section d'un cylindre le long de son axe longitudinal de droite à gauche latéralement avec une courbure en forme d'arc orientée dans le sens transversal-ventral, et
b) les éléments glissants supérieur et inférieur (11, 12) sont conçus avec une surface d'articulation (17) intérieure concave et la géométrie des concavités (17) est définie **en ce que** celle-ci comporte un évidement correspondant à une convexité (16) s'articulant de l'élément glissant central (13) et les concavités (17) sont fermées sur le côté supérieur et inférieur respectivement dorsalement, ventralement et latéralement des deux côtés par une bordure (14), et
c) les bordures (14) des éléments glissants (11, 12) s'articulant comportent un angle (angle d'ouverture, 21) s'ouvrant vers l'extérieur l'un par rapport à l'autre,
a. aucune inclinaison des éléments glissants (11, 12) l'un par rapport à l'autre n'étant possible dans le sens latéral, et
b. le mouvement maximal possible des éléments glissants (11, 12) dans le sens dorsoventral étant limité par une fermeture interstitielle des bordures (14) des éléments glissants (11, 12), et
d) la rotation des éléments glissants l'un par rapport à l'autre est limitée par la tolérance entre les convexités et les concavités à droite et à gauche latéralement des surfaces d'articulation cylindriques courbées transversalement.

3. Endoprothèse de disque intervertébral selon la revendication 2, dans laquelle les convexités (16) de l'élément glissant central (13) s'étendent respectivement sur tout le côté supérieur et inférieur ou dans laquelle les convexités sont entourées par une bordure (14).

4. Endoprothèse de disque intervertébral selon au moins une des revendications 1 à 3, dans laquelle une inclinaison des éléments glissants (11, 12) plus grande dans le sens ventral que dans le sens dorsal est possible.

5. Endoprothèse de disque intervertébral selon au moins une des revendications 1 à 4, dans laquelle une convexité cylindrique (16) comporte des rayons se réduisant de manière conique d'un côté le long de son axe longitudinal et dans laquelle la bordure (14) est adaptée à la diminution des rayons.

6. Endoprothèse de disque intervertébral selon au moins une des revendications 1 à 5, dans laquelle les rayons de courbure ventrale et dorsale des convexités courbées en forme d'arc (16) et des concavités (17) correspondantes sont identiques ou se différencient les uns des autres.

7. Endoprothèse de disque intervertébral selon au moins une des revendications 1 à 6, dans laquelle le mouvement maximal possible des éléments glissants (11, 12 et 13) est déterminé
a) par l'inclinaison dorsoventrale des éléments glissants (11, 12 et 13) l'un par rapport à l'autre, par le rayon de courbure ainsi que la hauteur de la convexité (16) et de la concavité (17) et la bordure correspondante (14), qui entoure une surface d'articulation ventralement et dorsalement, et
b) l'angle d'ouverture (21) entre les bordures (14) des éléments glissants (11, 12 et 13) voisins, celui-ci étant formé par les bordures (14) disposées de manière inclinée et/ou horizontalement des éléments glissants (11, 12 et 13) voisins, une fermeture interstitielle survenant lors du contact graduel des éléments glissants (11, 12 et 13), et
c) la rotation des éléments glissants par rapport à un axe vertical virtuel, par la/les courbure(s) en forme d'arc et la dimension de la/des courbure(s) en forme d'arc de la/des convexité(s) cylindrique(s) et de la/des concavité(s) correspondante(s) et la tolérance entre la convexité et la concavité respectivement à droite et à gauche à l'extrémité des surfaces d'articulation cylindriques.

8. Endoprothèse de disque intervertébral selon au moins une des revendications 1 à 7, dans laquelle l'élément glissant (11, 12 et 13) est conçu en une seule pièce.

9. Endoprothèse de disque intervertébral selon au moins une des revendications 1 à 7, dans laquelle au moins un élément glissant (11, 12 et 13) est composé d'au moins deux pièces liées l'une avec l'autre de manière fixe ou fixe, mais réversible, la/les convexité(s) (16) et/ou la/les concavité(s) (17) formant la pièce qui est reliée de manière fixe ou fixe, mais réversible, avec l'élément glissant (11, 12 et 13) correspondant, ou dans laquelle une/des convexité(s) (16) et/ou concavité(s) (17) comporte(nt) sur la base des moyens pour une liaison fixe ou fixe, mais réversible, avec l'élément glissant correspondant.

10. Endoprothèse de disque intervertébral selon au moins une des revendications précédentes, dans laquelle les éléments glissants (11, 12 et 13) et la/les convexité(s) (16) et/ou concavité(s) (17) ainsi que la bordure entourante sont composés du même matériau ou d'un matériau différent.

11. Endoprothèse de disque intervertébral selon au moins une des revendications suivantes, dans laquelle les surfaces des éléments glissants (11, 12 et 13) et/ou des pièces reliées les unes aux autres sont recouvertes de manière identique ou différente.

12. Endoprothèse de disque intervertébral selon les revendications 9 à 11, dans laquelle la liaison fixe ou fixe, mais réversible, entre les éléments glissants (11, 12 et 13) et la/les convexité(s) (16) ou concavité(s) (17) est formée par une liaison écrou/ressort, un rail de guidage et l'évidement correspondant, un mécanisme à déclic, un collage ou un vissage.

13. Endoprothèse de disque intervertébral selon au moins une des revendications 2 à 12, dans laquelle les éléments glissants supérieur et inférieur (11, 12) sont composés du même matériau ou recouverts de manière identique et l'élément glissant central (13) est fabriqué dans un autre matériau ou est recouvert de manière différente.

14. Endoprothèse de disque intervertébral selon au moins une des revendications précédentes, dans laquelle la hauteur maximale d'une convexité (16) est inférieure à un demi-cylindre et/ou la hauteur de la bordure (14) est réduite, dans laquelle la hauteur des convexités (16) sur le côté supérieur et inférieur est identique ou différente dans le cas d'une endoprothèse de disque intervertébral en trois parties.

15. Endoprothèse selon la revendication 2 ou au moins une des revendications précédentes s'y rapportant, dans laquelle les rayons de courbure des convexités (16) sur le côté supérieur et inférieur de l'élément glissant central (13) ainsi que les concavités correspondantes (17) sur l'élément glissant supérieur et inférieur (11, 12) sont identiques ou différentes.

16. Endoprothèse de disque intervertébral selon au moins une des revendications précédentes, dans laquelle l'angle d'ouverture maximal pour une fermeture interstitielle d'un côté des éléments glissants (11, 12 et 13) lors de l'extension ou de la flexion est compris entre 6 et 10 degrés, avec une tolérance de 3 degrés supplémentaires dans chaque direction.

17. Endoprothèse de disque intervertébral selon au moins une des revendications précédentes, dans laquelle la rotation autour d'un axe vertical central virtuel pour une courbure en forme d'arc orientée dans le sens ventral de la/des convexités (16) et de la/des concavités (17) entre les éléments glissants voisins (11, 12 et 13) est freinée, et un mouvement de rotation limité des éléments glissants (11, 12 et 13) par rapport à un axe vertical central virtuel est possible jusqu'à 3 degrés pour les vertèbres lombaires et jusqu'à 6 degrés pour les vertèbres cervicales de chaque côté, avec une tolérance de 2 degrés supplémentaires de chaque côté.

18. Endoprothèse de disque intervertébral selon au moins une des revendications précédentes, dans laquelle la/les convexités (16) et la/les concavités correspondantes (17) sont décalées de jusqu'à 4 mm de la section frontale dans le sens dorsal.

19. Endoprothèse de disque intervertébral selon au moins une des revendications précédentes, dans laquelle les bordures (14) des éléments glissants (11, 12 et 13) sont fermées ventralement, latéralement et dorsalement perpendiculairement vers l'extérieur, angulairement ailleurs, de manière courbée ou droite et courbée de manière associée et/ou angulairement.

20. Endoprothèse de disque intervertébral selon la revendication 2 ou au moins une des revendications précédentes s'y rapportant, dans laquelle les convexités de l'élément glissant central (13) sont conçues dans la zone latérale extérieure des deux côtés symétriquement ou asymétriquement, perpendiculairement, angulairement ailleurs, en forme de demi-sphère, de manière arrondie ou aplatie.

21. Endoprothèse de disque intervertébral selon la revendication 2 ou au moins une des revendications précédentes s'y rapportant, dans laquelle, pour fixer l'élément glissant central (13) contre une sortie en dehors de la prothèse lors de la fermeture interstitielle des trois éléments glissants (11, 12, 13), une butée fait partie de la bordure (14) de l'élément glissant central (13), la butée étant disposée en dehors de l'élément glissant (11, 12) supérieur et/ou inférieur, la butée étant plus haute au moins sur le côté inférieur et supérieur que la bordure (14) de l'élément glissant central (13).

22. Endoprothèse de disque intervertébral selon la revendication 2 ou au moins une des revendications précédentes s'y rapportant, dans laquelle pour une fixation supplémentaire de l'élément glissant central (13) contre une sortie en dehors de la prothèse lors de la fermeture interstitielle des trois éléments glissants (11, 12, 13), une butée fait partie de la bordure (14) de l'élément glissant central (13), la butée étant plus haute sur le côté supérieur et/ou inférieur que la bordure (14) de l'élément glissant central (13) et étant insérée à l'intérieur d'une rainure de la zone de bordure de l'élément glissant (11, 12) supérieur et/ou inférieur avec le jeu nécessaire pour le mouvement de glissement maximal des éléments glissants (11, 12 et 13).

23. Endoprothèse de disque intervertébral selon la revendication 2 ou au moins une des revendications précédentes s'y rapportant, dans laquelle pour une fixation supplémentaire d'un élément glissant central (13) avec une bordure (14) contre une sortie en dehors de la prothèse, lors de la fermeture interstitielle des trois éléments glissants (11, 12, 13), la bordure (14) de l'élément glissant central (13) est plus haute partiellement ou de manière continue en totalité à partir de la zone de passage de la convexité vers la périphérie et la bordure de l'élément glissant supérieur et/ou inférieur (11, 12) est plus plate dans la même mesure.

24. Endoprothèse de disque intervertébral selon la revendication 2 ou au moins une des revendications précédentes s'y rapportant, dans laquelle pour une fixation supplémentaire d'un élément glissant central (13) contre la sortie en dehors de la prothèse, lors de la fermeture interstitielle de tous les éléments glissants (11, 12, 13), les bordures extérieures des éléments glissants supérieur et/ou inférieur (11, 12) sont conçues totalement ou partiellement en forme de crochet, perpendiculairement, angulairement, courbées ou une combinaison de cela dans le sens de l'autre élément glissant extérieur.

25. Endoprothèse de disque intervertébral selon au moins une des revendications précédentes, dans laquelle les surfaces et la forme de la périphérie externe de l'élément glissant supérieur et inférieur (11, 12) sont identiques ou différentes et sont ainsi adaptées à la dimension correspondante du corps vertébral avec lequel elles sont reliées.

26. Endoprothèse de disque intervertébral selon au moins une des revendications précédentes, dans laquelle l'élément glissant supérieur et/ou inférieur (11, 12) est conçu en coupe frontale ou sagittale de sorte que les côtés extérieur et intérieur de l'élément glissant supérieur et/ou inférieur (11, 12) soient disposés parallèlement ou non l'un par rapport à l'autre.

27. Endoprothèse de disque intervertébral selon au moins une des revendications précédentes, dans laquelle une convexité d'une prothèse en deux parties (11, 12) et le côté supérieur et inférieur de l'élément glissant central (13) d'une prothèse en trois parties sont parallèles ou non par rapport à une horizontale et se trouvent donc dans un angle défini l'un par rapport à l'autre, la/les convexité(s) étant symétrique(s) ou asymétrique(s).

28. Endoprothèse de disque intervertébral selon au moins une des revendications précédentes, dans laquelle la courbure de la convexité (16) en forme d'arc dans le sens transversal - ventral est conçue symétriquement ou asymétriquement du centre vers les deux côtés latéraux.

29. Endoprothèse de disque intervertébral selon au moins une des revendications précédentes, dans laquelle la convexité (16) comporte une rupture au centre et la concavité (17) correspondante comporte une rupture sous forme de tige.

30. Endoprothèse de disque intervertébral selon au moins une des revendications précédentes, dans laquelle les éléments glissants supérieur et inférieur (11, 12) sont recouverts sur le côté extérieur de manière plane ou convexe et de manière bioactive ou sont en retrait et, pour l'ancrage primaire aux corps vertébraux, comportent des frottements de dents d'ancrage (20), lesquels sont disposées soit du sens dorsal au sens ventral de manière droite ou inclinée, soit sont disposées dans le sens latéral, des dents d'ancrage (20) se trouvant uniquement sur les côtés dans la ligne dorsale.

31. Endoprothèse de disque intervertébral selon au moins une des revendications précédentes, dans laquelle l'élément glissant supérieur et/ou inférieur (11, 12) comporte un moyen pour la mise en prise d'un instrument d'implantation ou d'explantation.

32. Endoprothèse de disque intervertébral selon au moins une des revendications précédentes, dans laquelle celle-ci comporte une largeur maximale (vue de face) de 14 à 48 mm, une profondeur maximale (coupe sagittale) de 11 à 35 mm et une hauteur maximale de 4 à 18 mm.

33. Endoprothèse de disque intervertébral selon au moins une des revendications précédentes, adaptée pour l'implantation de vertèbres lombaires, dans laquelle la périphérie extérieure de l'élément glissant supérieur et inférieur (11, 12) se réduit dans la vue transversale dans le sens ventral.

34. Endoprothèse de disque intervertébral selon au moins une des revendications 1 à 32 adaptée pour l'implantation dans les vertèbres cervicales, dans laquelle la périphérie extérieure de l'élément glissant supérieur et inférieur (11, 12) se réduit en vue transversale dans le sens dorsal.

35. Endoprothèse de disque intervertébral selon la revendication 33 ou 34, dans laquelle le rétrécissement de la périphérie extérieure de l'élément glissant supérieur et inférieur (11, 12) est conçu respectivement latéralement comme des courbures identiques ou asymétriquement.

36. Endoprothèse de disque intervertébral selon au moins une des revendications précédentes, dans laquelle les prothèses (11, 12 et 13) contiennent respectivement en dessous de la surface un ou plusieurs marquages visibles aux rayons X.
